(19) 

**Europäisches Patentamt**
**European Patent Office**
**Office européen des brevets**

(11) **EP 4 660 180 A1**

(12) **EUROPEAN PATENT APPLICATION**
published in accordance with Art. 153(4) EPC

(43) Date of publication:
**10.12.2025 Bulletin 2025/50**

(21) Application number: **24750229.7**

(22) Date of filing: **29.01.2024**

(51) International Patent Classification (IPC):
*C07C 211/63* (2006.01)   *C07C 303/40* (2006.01)
*C07C 311/48* (2006.01)   *H10K 30/40* (2023.01)
*H10K 30/50* (2023.01)   *H10K 30/86* (2023.01)
*H10K 50/135* (2023.01)   *H10K 85/50* (2023.01)
*H10K 85/60* (2023.01)

(52) Cooperative Patent Classification (CPC):
**C07C 311/48; C07C 211/04; C07C 211/05;**
**C07C 211/07; C07C 211/27; H10K 30/40;**
**H10K 85/50; H10K 85/60;** H10K 30/50;
H10K 50/135; Y02E 10/549

(86) International application number:
**PCT/JP2024/002706**

(87) International publication number:
**WO 2024/162280 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.01.2023 JP 2023011694**
**21.06.2023 JP 2023102060**

(71) Applicant: **National Institute of Advanced
Industrial
Science and Technology
Chiyoda-ku
Tokyo 100-8921 (JP)**

(72) Inventors:
• **NISHIMURA, Naoyuki**
**Tsukuba-shi, Ibaraki 305-8560 (JP)**
• **TACHIBANA, Hiroaki**
**Tsukuba-shi, Ibaraki 305-8560 (JP)**
• **MURAKAMI, Takurou**
**Tsukuba-shi, Ibaraki 305-8560 (JP)**

(74) Representative: **Hasegawa, Kan
Patentanwaltskanzlei Hasegawa
Untere Hauptstraße 56
85354 Freising (DE)**

(54) **IONIC COMPOUND, HOLE TRANSPORT MATERIAL CONTAINING IONIC COMPOUND,
PEROVSKITE SOLAR CELL CONTAINING HOLE TRANSPORT MATERIAL, METHOD FOR
PRODUCING IONIC COMPOUND, METHOD FOR PRODUCING HOLE TRANSPORT
MATERIAL, AND METHOD FOR PRODUCING PEROVSKITE SOLAR CELL**

(57) The present invention provides an ionic compound including a molecular cation and a molecular anion, wherein the molecular cation includes at least one organic ammonium selected from the group consisting of a primary organic ammonium, a secondary organic ammonium, and a tertiary organic ammonium, wherein the organic ammonium has an organic moiety having 1 to 20 carbon atoms, and wherein the molecular anion includes a fluorine-containing bis(sulfonyl)imide.

EP 4 660 180 A1

**Description**

TECHNICAL FIELD

The present disclosure relates to an ionic compound, a hole-transporting material containing the ionic compound and a perovskite solar cell including the hole-transporting material, as well as methods of producing the ionic compound, the hole-transporting material and the perovskite solar cell.

BACKGROUND ART

Ionic compounds are compounds containing anions and cations. The ionic compounds have properties such as non-volatility and non-flammability as well as electrical conductivity, and are used in various applications, such as additives and reagents for electric materials (electronic materials), solvents for organic synthesis, and the like. When used for electronic materials, for example, a technique of adding an ionic compound as an additive (dopant) to an organic electronic material has been performed for the purpose of changing the electrical conductivity.

Typical examples of devices using organic electronic materials include perovskite solar cells. A perovskite solar cell is known as a solar cell in which a material containing an organic cation such as formamidinium, an inorganic cation such as lead cation, and a halogen anion and the like, referred to as a "perovskite material", is used for a photoabsorption layer (Patent Documents 1 and 2). This perovskite layer is hydrophilic, and is known to form a hydrate (Non-patent Document 1). Such a solar cell is drawing attention as a next-generation solar cell, because the solar cell can be produced by a wet coating method, and is capable of achieving a performance equal to or higher than that of a crystalline silicon solar cell. In particular, such a solar cell has a higher open circuit voltage as compared to a conventional crystalline silicon solar cell, and is expected to be used suitably in applications that require a high electromotive force, such as, for example, an application as a power supply for water electrolysis. In a perovskite solar cell, an organic electronic material is often provided so as to be bonded to the perovskite layer, as a hole-transporting material. The hole-transporting material gives the anisotropy of charge transfer, making it possible to realize a high-performance perovskite solar cell. To the hole-transporting material, a dopant is added for the purpose of improving the hole mobility.

Adding such a dopant to the hole-transporting material facilitates the movement of the holes, making it possible to obtain a high photoelectric conversion efficiency. Typical examples of the ionic compound as a dopant include Li-TFSI, which is composed of a lithium (Li) cation and bis(trifluoromethanesulfonyl)imide (TFSI) anion (Patent Documents 1 and 2).

Further, Non-patent Document 2 discloses the use of TBA-TFSI in which tetrabutylammonium (TBA) cation is included as the cation, as a dopant.

Non-patent Document 3 discloses a technique of adding 4-tert-butylpyridine to a dopant for the hole-transporting material. 4-Tert-butylpyridine has a relatively low boiling point, and is known to adversely affect the heat resistance of the resulting solar cell.

In addition to additives for electric materials, ionic compounds are also used as base materials for electric materials, electrolytes, solvents and precursors for material synthesis, gas adsorbents, and the like. In particular, ionic compounds having a melting point of 100°C or lower are known as ionic liquids. Ionic liquids have a high chemical stability such as non-volatility and non-flammability, are easy to handle at a low temperature, and also have properties such as electrical conductivity. Therefore, ionic compounds are suitable for the above-described base materials for electric materials, and the like, and there is a potential possibility for further expansion of applications.

As an anion of an ionic compound, a molecular anion including a fluorine-containing bis(sulfonyl)imide, such as the TFSI anion described above, has a high electronegativity and is known as a promising anion of an ionic liquid. Further, examples of known cations include quaternary ammonium-based cations, imidazole-based cations, and pyridine-based cations.

PRIOR ART DOCUMENTS

PATENT DOCUMENTS

Patent Document 1: WO 2021/131428
Patent Document 2: JP 2019-96891 A

NON-PATENT DOCUMENTS

Non-patent Document 1: P.V. Kamat et al., J. Am. Chem. Soc. 2015, 137, 1530-1538
Non-patent Document 2: Jinbao Zhang et al., ACS Energy Lett. 2018, 3, 1677-1682
Non-patent Document 3: Shen Wang et al., Nano Lett. 2016, 16, 5594-5600

SUMMARY OF INVENTION

TECHNICAL PROBLEM

While ionic compounds are widely used in applications such as electric materials, reagents and solvents for organic synthesis, as described above, improvements in various properties are required. In particular, cations to be included in the ionic compounds are required to have a better reactivity. Specifically, the cations are required to have a better nucleophilic reactivity, a better electrophilic reactivity and/or the like, and more specifically, a higher cationicity and a larger electron orbital spread, in various applications.

For example, a further improvement in performance utilizing ionic compounds is required for perovskite solar cells. In particular, a further improvement in open circuit voltage, which is a characteristic of a perovskite solar cell, is desired. In addition, a higher durability and a longer service life as a solar cell are required. However, the present inventors arrived at the fact that a quaternary ammonium or pyridine, which has been used as a conventional additive, has a low cationicity. Specifically, it is thought that the cation of imidazole or pyridine is a cation with sp2 electron orbitals, and thus has an electron orbital spread smaller than that of a cation with sp3 orbitals, thereby limiting the effect thereof.

Further, a higher cationicity and a larger electron orbital spread are required, as a catalyst, a photocatalyst, a gas adsorbent and a solvent, in order to more effectively exhibit catalytic ability, photocatalytic activity, gas adsorption capacity or ability to dissolve substances.

The present disclosure provides an ionic compound containing a cation having an excellent reactivity, a method of producing the ionic compound, a hole-transporting material containing such an ionic compound, a method of producing the hole-transporting material, a perovskite solar cell including the hole-transporting material, and a method of producing the perovskite solar cell.

SOLUTION TO PROBLEM

The present inventors have found out that an ionic compound which includes a cation including an organic ammonium having an organic moiety having 1 to 20 carbon atoms, and a molecular anion including a fluorine-containing bis(sulfonyl)imide, has an excellent reactivity, thereby completing the present disclosure. Specifically, the present disclosure is as described below.

The present disclosure provides an ionic compound including a molecular cation and a molecular anion,

wherein the molecular cation includes at least one organic ammonium selected from the group consisting of a primary organic ammonium, a secondary organic ammonium, and a tertiary organic ammonium,
wherein the organic ammonium has an organic moiety having 1 to 20 carbon atoms, and
wherein the molecular anion includes a fluorine-containing bis(sulfonyl)imide.

Further, the present disclosure provides a hole-transporting material containing the ionic compound described above.

Still further, the present disclosure provides a perovskite solar cell including:

a perovskite layer; and
a hole-transporting layer formed on the perovskite layer,

wherein the hole-transporting layer contains the hole-transporting material described above.

Yet still further, the present disclosure provides a method of producing the ionic compound described above, the method including the steps of:

ion-exchanging a halide of an organic ammonium having an organic moiety having 1 to 20 carbon atoms, with an alkali metal salt of a fluorine-containing bis(sulfonyl)imide; and
extracting the ionic compound obtained by the ion exchange.

Yet still further, the present disclosure provides a method of producing the ionic compound described above, the method including the step of neutralizing an organic amine having an organic moiety having 1 to 20 carbon atoms, with an acid of a fluorine-containing bis(sulfonyl)imide.

Yet still further, the present disclosure provides a method of producing a hole-transporting material, the method including the step of mixing the ionic compound described above, and a raw material compound of the hole-transporting material.

Yet still further, the present disclosure provides a method of producing a perovskite solar cell by layering an electron-transporting layer, a perovskite layer and a hole-transporting layer between an electrically conductive substrate and an electrode,

> wherein the method of producing the perovskite solar cell includes the step of forming the hole-transporting layer, and
> wherein the step of forming the hole-transporting layer includes the step of forming a film on the perovskite layer, using the hole-transporting material described above.

ADVANTAGEOUS EFFECTS OF INVENTION

The present disclosure can provide an ionic compound containing a cation having an excellent reactivity, a method of producing the ionic compound, a hole-transporting material containing such an ionic compound, a method of producing the hole-transporting material, a perovskite solar cell including the hole-transporting material, and a method of producing the perovskite solar cell.

BRIEF DESCRIPTION OF DRAWINGS

> FIG. 1 is a schematic diagram showing one example of the basic configuration of a perovskite solar cell.
> FIG. 2 shows a $^1$H-NMR spectrum of n-octylammonium TFSI (Example 1).
> FIG. 3 shows a $^{19}$F-NMR spectrum of n-octylammonium TFSI (Example 1).
> FIG. 4 shows a $^1$H-NMR spectrum of n-octylammonium TFSI (Example 2).
> FIG. 5 shows a $^1$H-NMR spectrum of n-dodecylammonium TFSI (Example 3).
> FIG. 6 shows a $^1$H-NMR spectrum of n-butylammonium TFSI (Example 4).
> FIG. 7 shows a $^1$H-NMR spectrum of ethylammonium TFSI (Example 5).
> FIG. 8 shows a $^1$H-NMR spectrum of 2-phenylethylammonium TFSI (Example 6).
> FIG. 9 shows a $^1$H-NMR spectrum of n-methyl-n-octylammonium TFSI (Example 7).
> FIG. 10 shows a $^1$H-NMR spectrum of n-octylammonium FSI (Example 8).
> FIG. 11 shows a $^1$H-NMR spectrum of methylammonium TFSI (Example 9).
> FIG. 12 shows a $^1$H-NMR spectrum of n-octylammonium PFSI (Example 18).
> FIG. 13 shows a $^1$H-NMR spectrum of n-octylammonium NFSI (Example 19).
> FIG. 14 shows a $^1$H-NMR spectrum of n,n-dimethyl-n-octylammonium TFSI (Example 20).

DESCRIPTION OF EMBODIMENTS

The present disclosure will be described below in detail. However, the present disclosure is in no way limited to the following description.

The ionic compound according to an embodiment of the present disclosure is an ionic compound including a molecular cation and a molecular anion,

> wherein the molecular cation includes at least one organic ammonium selected from the group consisting of a primary organic ammonium, a secondary organic ammonium, and a tertiary organic ammonium,
> wherein the organic ammonium has an organic moiety having 1 to 20 carbon atoms, and
> wherein the molecular anion includes a fluorine-containing bis(sulfonyl)imide.

In cases where a numerical range is described in the present specification, the numerical range is defined to include the upper limit and the lower limit of the range. In other words, the description "XX or more and YY or less" or "from XX to YY" used to indicate a numerical range refers to a numerical range that includes the lower limit and the upper limit which are the end points of the range, unless otherwise defined. In cases where the numerical ranges are described in stages, the upper limit and the lower limit of each numerical range can be combined arbitrarily.

By adopting the configuration described above, the ionic compound according to the present embodiment shows an excellent reactivity due to the molecular cation and an effect due to the molecular anion. Such effects work suitably in applications that utilize an excellent reactivity due to the molecular cation and/or the effect due to the molecular anion, such as various applications described above. For example, the present ionic compound can be used as an additive for a hole-transporting material in a perovskite solar cell, which is suitable for enhancing the performance and/or the durability of the solar cell. While the inventors do not wish to be bound by any particular theory, the mechanism for enhancing the performance and/or the durability of the perovskite solar cell is assumed to be as follows.

First, at least one organic ammonium cation selected from the group consisting of a primary organic ammonium cation, a secondary organic ammonium cation and a tertiary organic ammonium cation, which is derived from the ionic compound according to the present embodiment that has been added to the hole-transporting material as a dopant, and which is a cation having an excellent reactivity, spontaneously reacts with the surface of the perovskite layer, to decrease the defects on the perovskite surface and/or to improve the electrical bonding interface with the hole-transporting material, thereby improving the solar cell properties such as the open circuit voltage. Further, the molecular anion including a fluorine-containing bis(sulfonyl)imide, which is derived from the ionic compound according to the present embodiment, extracts electrons from the hole-transporting material to cause oxidation, making it possible to improve the hole mobility in the hole-transporting material. In particular, the synergistic effect due to the combination of the above-described cation and anion is assumed to occur. Since the molecular anion including a fluorine-containing bis(sulfonyl)imide has a high ionicity, the anion reacts effectively with the hole-transporting material. As a result, the association between the organic ammonium cation and the anion described above becomes weak, making it possible to allow the reaction between the organic ammonium cation and the perovskite to proceed spontaneously. In addition, the negative charge of the anion becomes independent as a result of the reaction between the organic ammonium cation and the perovskite, making it possible to extract electrons more effectively from the hole-transporting material as compared to when the anion coexists with the cation. Either one or the synergistic effect of both of these is thought to be responsible for suitably improving the performance of the resulting solar cell.

Further, the fact that the above-described organic ammonium cation, which is a molecular cation having an excellent reactivity, spontaneously reacts with the surface of the perovskite layer, to decrease the defects on the perovskite surface and/or to improve the electrical bonding interface with the hole-transporting material, is advantageous in that the strict control of the production environment can be alleviated. In a conventional process of depositing a hole-transporting layer on a perovskite layer, the perovskite surface is exposed to the atmosphere, and reacts with a solvent, or with oxygen molecules particularly in the air atmosphere, to form defects on the perovskite surface, and therefore, a strict control of the production environment, such as producing a perovskite solar cell in nitrogen, is required. However, the fact that the above-described organic ammonium cation, which is a cation having an excellent reactivity, spontaneously reacts with the surface of the perovskite layer during the deposition of the hole-transporting material, eliminates the risk that the perovskite surface whose defects have been reduced is directly exposed to the atmosphere. This makes the perovskite solar cell less susceptible to the influence by the production environment, enabling the alleviation of the need to control the production environment. As a result, the production of a perovskite solar cell having a relatively high performance can be suitably performed even in the air atmosphere, not in an inert atmosphere such as a nitrogen atmosphere.

(Organic Ammonium Cation)

At least one organic ammonium cation (hereinafter, also referred to as "organic ammonium") selected from the group consisting of a primary organic ammonium cation (hereinafter, also referred to as "primary organic ammonium"), a secondary organic ammonium cation (hereinafter, also referred to as "secondary organic ammonium") and a tertiary organic ammonium cation (hereinafter, also referred to as "tertiary organic ammonium") has an excellent reactivity. In addition, the raw materials of such organic ammonium cations are relatively easily available at low cost and are relatively safe. The primary organic ammonium cation is represented by the following Formula (1), the secondary organic ammonium cation is represented by the following Formula (5), and the tertiary organic ammonium cation is represented by the following Formula (6).

$$RNH_3^+ \qquad (1)$$

(In Formula (1), R represents an organic moiety having 1 to 20 carbon atoms.)

$$R_2NH_2^+ \qquad (5)$$

(In Formula (5), each R independently represents an organic moiety having 1 to 20 carbon atoms.)

$$R_3NH^+ \qquad (6)$$

(In Formula (6), each R independently represents an organic moiety having 1 to 20 carbon atoms.)

The organic ammonium is more preferably the primary organic ammonium, from the viewpoint of obtaining a better reactivity.

The degree of substitution of the organic ammonium is preferably small, from the viewpoint of obtaining an excellent reactivity. In the case of using the ionic compound as an additive for a perovskite solar cell, in particular, the degree of substitution of the organic ammonium is preferably small, from the viewpoint of obtaining an excellent reactivity with the perovskite. From this point of view, specifically, the organic ammonium is preferably at least one organic ammonium selected from the group consisting of a primary organic ammonium and a secondary organic ammonium ion, and most preferably a primary organic ammonium. On the other hand, the degree of substitution of the organic ammonium is preferably large, from the viewpoint of deceasing the viscosity of the resulting ionic liquid, making it advantageous when mixed with and diffused into another material(s). From this point of view, the organic ammonium is preferably at least one organic ammonium selected from the group consisting of a secondary organic ammonium and a tertiary organic ammonium ion, and most preferably a tertiary organic ammonium.

The organic ammonium cation has an organic moiety having 1 to 20 carbon atoms. The organic ammonium cation preferably has an organic moiety having 2 to 20 carbon atoms. The number of carbon atoms in the organic moiety of the organic ammonium cation is 20 or less, and more preferably 12 or less, for example, from the viewpoint that the bulkiness of the organic ammonium cation can be decreased and the density of the resulting ionic compound itself can be increased, and the viewpoint that the ionic compound can be added more densely and effectively on the basis of on the number of molecules, as a dopant, a catalyst, a gas adsorbent, a solvent and/or the like.

Further, the number of carbon atoms in the organic moiety of the organic ammonium cation is 1 or more, more preferably 2 or more, and still more preferably 4 or more, from the viewpoint that the melting point of the resulting ionic compound can be more easily decreased, making it advantageous in the application as a liquid. The number of carbon atoms in the organic moiety of the organic ammonium cation may be, for example, from 2 to 12, from 4 to 20, or from 4 to 12.

In the case of using the ionic compound according to the present embodiment as a dopant for a hole-transporting material in a perovskite solar cell, in particular, the number of carbon atoms in the organic moiety of the organic ammonium cation is preferably 7 or less, from the viewpoint of decreasing the dopant concentration dependence, making it advantageous in enlargement of the cell area. From this point of view, the number of carbon atoms in the organic moiety of the organic ammonium cation is more preferably 4 or less, and still more preferably 2 or less. In other words, from such a point of view, the number of carbon atoms in the organic moiety of the organic ammonium cation may be, for example, from 1 to 7, from 1 to 4, or from 1 to 2 or less. In particular, the number of carbon atoms is particularly preferably from 1 to 2.

From the viewpoint of more effectively hydrophobizing the perovskite surface, making it advantageous in improving the durability, on the other hand, the number of carbon atoms in the organic ammonium cation is preferably 9 or more, and more preferably 12 or more. In other words, from such a point of view, the number of carbon atoms in the organic moiety of the organic ammonium cation may be, for example, from 9 to 20, or from 12 to 20.

At the same time, the number of carbon atoms in the organic ammonium cation is also preferably 1, from the viewpoint that there is a small decrease in performance even when diffused from the perovskite surface into the bulk, because the cation can be the same as the A site of a three-dimensional perovskite, the viewpoint that the ionic compound can be suitably used as an additive for a perovskite material, the viewpoint that the crystal growth of a perovskite material can be more easily promoted, and/or the viewpoint that it is advantageous in surface stabilization at the perovskite grain boundaries.

The organic moiety having 1 to 20 carbon atoms may be, for example, an aliphatic or aromatic moiety.

The aliphatic moiety may be, for example, a saturated or unsaturated aliphatic hydrocarbon. In other words, the organic moiety of the ammonium may contain a saturated hydrocarbon or an unsaturated hydrocarbon. The saturated hydrocarbon and the unsaturated hydrocarbon may each be cyclic, linear or branched. Of these, a saturated hydrocarbon (such as alkyl) is preferred in the case of using the ionic compound as a dopant for the hole-transporting material, from the viewpoint that the carbon chain has a high degree of freedom and thus facilitates the reaction with the perovskite surface. The saturated hydrocarbon may be, for example, a linear saturated hydrocarbon or a cyclic saturated hydrocarbon. The saturated hydrocarbon is preferably a linear saturated hydrocarbon, because of its ability to precisely react with the perovskite surface. The same can be said in the case of other applications, because of its high cationicity, a large electron orbital spread and the like.

The aromatic moiety may be, for example, an aromatic hydrocarbon or an aromatic heterocyclic ring. In other words, the organic moiety of the ammonium may contain an aromatic hydrocarbon or an aromatic heterocyclic ring. The organic moiety of the organic ammonium preferably contains an aromatic moiety, from the viewpoint that the arrangement of the organic ammonium on the perovskite can be controlled by the $\pi$-$\pi$ interaction, and the viewpoint that it is advantageous in improving the adhesion between the hole-transporting layer and the perovskite layer. While the inventors do not wish to be bound by any particular theory, the mechanism for improving the adhesion is assumed to be as follows. It is assumed that the adhesion between the aromatic moiety which can be included in the hole-transporting material, and the aromatic moiety included in the organic moiety of the organic ammonium adsorbed on the perovskite surface, is improved through the $\pi$-$\pi$ interaction. Further, it is assumed that the induced dipole in a hole-transporting material solution has an affinity with the induced dipole of the aromatic moiety included in the organic moiety of the organic ammonium adsorbed on the perovskite surface, and the wettability to the perovskite surface provided by the hole-transporting material solution is improved, to interfere with the formation of pores, resulting in a dense deposition of the hole-transporting layer on the perovskite layer. The organic moiety containing an aromatic hydrocarbon or an aromatic heterocyclic ring may be, for example, an aryl group, a heteroaryl group, a biphenyl group, a thienyl group, a pyridyl group, a pyrrolyl group or a furyl group. Of these, an aryl group is preferred, from the viewpoint that the arrangement of the organic ammonium on the perovskite can be effectively controlled, and the viewpoint that it is advantageous in effectively improving the adhesion between the hole-transporting layer and the perovskite layer.

The organic moiety having 1 to 20 carbon atoms may contain, for example, an alkyl group, an alkenyl group, an alkynyl group, a cycloalkyl group or the like. Specifically, examples of the alkyl group include methyl group, ethyl group, n-propyl group, isopropyl group, n-butyl group, isobutyl group, sec-butyl group, t-butyl group, neopentyl group, n-hexyl group, n-octyl group, n-dodecyl group and cyclohexyl group. Examples of the alkenyl group include vinyl group, 1-propenyl group, 2-propenyl group, isopropenyl group, 1-butenyl group, 2-butenyl group, 3-butenyl group, 1-octenyl group, 1-decenyl group and 1-octadecenyl group. Examples of the alkynyl group include ethynyl group, 1-propynyl group, 2-propynyl group, 1-butynyl group, 2-butynyl group, 3-butynyl group, 1-octynyl group, 1-decynyl group, 1-octadecynyl group, phenyl group, phenylmethyl group, phenylethyl group, phenylpropyl group, phenylbutyl group and trifluoroethyl group. Of these, the organic moiety may more preferably contain a methyl group, an ethyl group, a n-butyl group, a n-octyl group or a n-dodecyl group, from the viewpoint of obtaining an excellent reactivity with the perovskite surface.

The organic moiety may contain a functional group other than the organic ammonium. The functional group may be, for example, an oxygen-containing group, a nitrogen-containing group, or a sulfur-containing group. The organic moiety may contain a plurality of functional groups. In other words, the organic moiety of the organic ammonium may contain at least one functional group selected from the group consisting of an oxygen-containing group, a nitrogen-containing group, and a sulfur-containing group. In cases where the organic moiety contains a functional group, the number of carbon atoms (from 1 to 20) in the organic moiety is the number including that of the carbon atoms contained in the functional group. In cases where the organic moiety contains a carboxy group (COOH) as the functional group, for example, the number of carbon atoms contained in the COOH is included in the number of carbon atoms in the organic moiety.

The oxygen-containing group may specifically be, for example, a hydroxy group, an alkoxy group, an aryloxy group, an ester group, an acyl group, a carboxy group, a carbonyl group, or an epoxy group. Of these, the oxygen-containing group is preferably a hydroxy group, or a carboxy group.

The nitrogen-containing group may specifically be, for example, an amino group, an imino group, an amide group, an imide group, a hydrazino group, a hydrazono group, a nitro group, a nitroso group, a cyano group, an isocyano group, a cyanate ester group, an amidino group, a diazo group, a secondary ammonium, a tertiary ammonium, a pyridyl group, or an imidazolyl group. Of these, the nitrogen-containing group is preferably a secondary ammonium or a tertiary ammonium. Further, it is preferred that the organic moiety does not contain a quaternary ammonium, from the viewpoint of the reactivity.

The sulfur-containing group may be, for example, a thiol group or a sulfo group. Of these, the sulfur-containing group is preferably a thiol group.

The organic moiety of the organic ammonium cation can contain a halogen atom. Examples of the halogen atom include fluorine atom, chlorine atom, bromine atom and iodine atom. The organic moiety of the organic ammonium cation preferably contains a halogen atom, from the viewpoint that the nucleophilicity of the organic ammonium cation can be enhanced utilizing the electron-withdrawing properties of the halogen atom. Further, the organic moiety more preferably contains a fluorine atom, from the viewpoint that the surface energy of the ionic compound according to the present embodiment, and/or of a compound to which the ionic compound has been added, can be decreased.

More specifically, the organic moiety of the organic ammonium may be, for example, one which contains a fluorocarbon containing a halogen atom such as a fluorine atom. For example, the organic moiety of the organic ammonium may be, for example, one which contains a fluorocarbon, and in which the carbon atom bonded to the nitrogen atom of the organic ammonium has a $CH_2$ structure, or one (such as $CF_3$, $C_2F_5$ or the like) which contains a perfluoroalkyl group. The term "bond or to bond" as used herein refers to a chemical bond, such as a covalent bond.

In particular, it is preferred that the carbon atom bonded to the nitrogen atom of the organic ammonium cation have a $CH_2$ structure, and that the remaining carbon chain be perfluoroalkyl. This is because, when the carbon atom bonded to the nitrogen atom of the organic ammonium cation has a $CH_2$ structure, the reaction of the fluorine atoms in the perfluoroalkyl with the protons of the ammonium is inhibited, making it possible to stabilize the perfluoroalkyl. Such a structure may be, for example, $C_nF_{2n+1}CH_2NH_3^+$ (wherein n represents an integer of 1 or more), and may specifically be $C_4F_9CH_2NH_3^+$.

From the viewpoint of reducing undesired side reactions, an embodiment in which the above-described organic ammonium cation does not contain a functional group other than the organic ammonium, is also one of the preferred embodiments. In particular, the organic moiety is preferably a linear alkyl group which does not contain a functional group and which has 1 to 12 carbon atoms, and more preferably a linear alkyl group which does not contain a functional group and which has 2 to 12 carbon atoms. Further, the organic moiety is still more preferably a linear alkyl group which does not contain a functional group and which has 4 to 12 carbon atoms.

Examples of the cation of the ionic compound according to the present embodiment include methylammonium, ethylammonium, propylammonium, butylammonium, pentylammonium, hexylammonium, heptylammonium, octylammonium, nonylammonium, decylammonium, undecylammonium, dodecylammonium, anilinium, methylphenylammonium, phenylethylammonium, phenylpropylammonium, and phenylbutylammonium. When the ionic compound according to the present embodiment is used as a dopant for a hole-transporting material in a perovskite solar cell, the cation of the ionic compound is preferably methylammonium, ethylammonium, propylammonium, butylammonium, pentylammonium, hexylammonium, heptylammonium, octylammonium, nonylammonium, decylammonium, undecylammonium, dodecylammonium, methylphenylammonium, phenylethylammonium, phenylpropylammonium or phenylbutylammonium, from the viewpoint of obtaining an excellent reactivity between the organic ammonium and the perovskite. From the viewpoint of decreasing the dopant concentration dependence, making it advantageous in enlargement of the cell area, the cation of the ionic compound is more preferably methylammonium, ethylammonium, propylammonium, butylammonium, pentylammonium, hexylammonium or heptylammonium, and still more preferably methylammonium, ethylammonium, propylammonium, or butylammonium. From the viewpoint of more effectively hydrophobizing the perovskite surface, making it advantageous in improving the durability, the cation of the ionic compound is more preferably nonylammonium, decylammonium, undecylammonium or dodecylammonium, and still more preferably decylammonium, undecylammonium, or dodecylammonium. From the viewpoint that the arrangement of the organic ammonium on the perovskite can be controlled by the $\pi$-$\pi$ interaction, and the viewpoint that it is advantageous in improving the adhesion between the hole-transporting layer and the perovskite layer, the cation of the ionic compound is more preferably methylphenylammonium, phenylethylammonium, phenylpropylammonium or phenylbutylammonium, and still more preferably phenylethylammonium such as 2-phenylethylammonium.

(Molecular Anion Including Fluorine-containing Bis(sulfonyl)imide)

The molecular anion includes a fluorine-containing bis(sulfonyl)imide. The fluorine-containing bis(sulfonyl)imide is represented by the following Formula (2).

$$RfSO_2N^-SO_2Rf \qquad (2)$$

(In Formula (2), each Rf independently represents a fluorine atom, or a fluorine-containing alkyl group having 1 to 10 (preferably from 1 to 3) carbon atoms.)

The fluorine-containing bis(sulfonyl)imide anion has two sulfonyl groups. A functional group such as a fluorine atom or a fluorocarbon may be bonded to each of the sulfonyl groups. The functional groups bonded to the two sulfonyl groups may be the same as, or different from, each other, and may each be cyclic. In the case of using the ionic compound as a dopant for the hole-transporting material, for example, the functional groups bonded to the two sulfonyl groups are preferably the same, from the viewpoint of allowing the electron withdrawal from the hole-transporting material to proceed effectively. More specific examples of the fluorine-containing bis(sulfonyl)imide include those having a perfluoroalkyl group, such as bis(fluorosulfonyl)imide (FSI), bis(trifluoromethanesulfonyl)imide (TFSI), bis(pentafluoroethanesulfonyl) imide (PFSI) and bis(nonafluorobutanesulfonyl)imide (NFSI). Of these, bis(trifluoromethanesulfonyl)imide is preferred, because of its high electronegativity and a high affinity with a solvent. In the case of using the ionic compound as a dopant for the hole-transporting material, for example, bis(trifluoromethanesulfonyl)imide is preferred from the viewpoint of allowing the electron withdrawal from the hole-transporting material to proceed effectively. From the viewpoint of deceasing the viscosity of the resulting ionic liquid, making it advantageous when mixed with and diffused into another material(s), the bis(fluorosulfonyl)imide anion is preferred. Further, bis(pentafluoroethanesulfonyl)imide or bis(nonafluorobutanesulfonyl)imide is preferred, because it is advantageous in hydrophobizing the hole-transporting material, in addition to hydrophobizing the perovskite layer.

The combination of the organic ammonium cation and the molecular anion is not particularly limited. However, a combination that allows the resulting ionic compound to be an ionic liquid is preferred, because the ionic compound can be used in an application as a liquid. In the case of using the ionic compound as an additive, such a combination is preferred, because the time required for dissolving the compound in a solvent can be shortened as compared to that in the form of a solid, making it industrially advantageous. The ionic liquid is an ionic compound having a melting point of 100°C or lower, and the definition thereof also includes an ionic compound which is liquid at normal temperature (from 5 to 35°C). Such an ionic liquid is non-volatile despite being a liquid and is non-flammable, has an excellent thermal stability, is easy to handle, and can be used under mild conditions. Therefore, the ionic liquid is suitable, not only for an electric material, but also as a gas adsorbent, a solvent for separation and extraction, or a reaction solvent. An ionic liquid which is liquid at normal temperature is preferred, because it can be easily used as an ionic liquid.

For example, when the number of carbon atoms in the organic moiety of the organic ammonium cation is from 4 to 12, and when the molecular anion is any one of FSI, TFSI and PFSI, it is advantageous because the resulting ionic compound tends to have a melting point equal to or lower than normal temperature, and is suitable because the compound can be easily used as an ionic liquid. For example, n-butylammonium TFSI, n-octylammonium TFSI, n-dodecylammonium TFSI, n-methyl-n-octylammonium TFSI, n,n-dimethyl-n-octylammonium TFSI, n-octylammonium FSI, n-octylammonium PFSI or the like is liquid at normal temperature, and thus is preferred as the ionic compound. The description such as "n-butylammonium TFSI" as used herein indicates that it is an ionic compound including a molecular cation and a molecular anion, in which the molecular cation is n-butylammonium and the molecular anion is TFSI. In other words, the "n-butylammonium TFSI" indicates that it is a salt of n-butylammonium and TFSI.

When the ionic compound according to the present embodiment is the ionic liquid described above, the ionic liquid preferably has a low viscosity, because it is advantageous when mixed with and diffused into another material(s). In the case of using the ionic compound as a dopant for the hole-transporting material, in particular, the ionic compound preferably has a low viscosity, because it is advantageous in quickly mixing the hole-transporting material and the ionic compound.

The viscosity of the ionic compound is not particularly limited. However, the viscosity may be from 1 to 2,000 m Pa·s, preferably from 10 to 600 m Pa·s, and more preferably 15 to 400 m Pa·s, under the condition of 25°C. The measurement of the viscosity is performed by the method to be described later. Specifically, the viscosity is measured using a Type E viscometer (TV-25, manufactured by Toki Sangyo Co., Ltd.) under the temperature condition of 25°C.

The ionic compound according to the present embodiment can contain an alkali metal cation. The concentration of the alkali metal contained in the ionic compound is preferably 100 ppm by mass or less, and more preferably 10 ppm by mass or less, because the reaction of the organic ammonium included in the ionic compound is not inhibited. In cases where the ionic compound is an ionic liquid, on the other hand, the concentration of the alkali metal contained in the ionic compound is preferably 1 ppb by mass or more, more preferably 10 ppb by mass or more, and still more preferably 100 ppb by mass or more, because it is advantageous in decreasing the viscosity. The alkali metal cation is preferably at least one selected from the group consisting of lithium cation, sodium cation and potassium cation, and most preferably lithium cation, because such a cation has a small ion radius, and is advantageous in dispersion.

The ionic compound can be produced by the ion exchange method or the acid-base neutralization method to be described below.

In the ion exchange method, the ionic compound can be easily produced by: ion-exchanging a halide of an organic ammonium having an organic moiety having 1 to 20 carbon atoms, with an alkali metal salt of a fluorine-containing bis(sulfonyl)imide, and then undergoing an extraction step. In other words, the method of producing the ionic compound preferably includes the steps of: ion-exchanging a halide of such an organic ammonium, with an alkali metal salt of a fluorine-containing bis(sulfonyl)imide; and extracting the ionic compound obtained by the ion exchange. The use of the ion exchange method is one of the preferred embodiments, because the anion raw material of the ionic compound according to the present embodiment used in the ion exchange method has better storage properties as compared that in the neutralization method to be described later. Specifically, the ion exchange method is preferred from the viewpoint that the alkali metal salt of the fluorine-containing bis(sulfonyl)imide to be used in the ion exchange method is more stable, as compared to the acid of the fluorine-containing bis(sulfonyl)imide to be used in the neutralization method, and thus excellent storage properties can be obtained. Further, the ion exchange method is preferred also from the viewpoint that the alkali metal can be easily incorporated into the ionic compound, making it easier to decrease the viscosity of the ionic liquid.

Examples of the halogen ion contained in the halide of the organic ammonium include fluorine ion, chlorine ion, bromine ion and iodine ion. Of these, the halogen ion is preferably a chlorine ion. A halide of any of the organic ammoniums described in the section of the organic ammonium described above can be used as the halide of the organic ammonium.

Examples of the alkali metal ion contained in the alkali metal salt of the fluorine-containing bis(sulfonyl)imide include lithium ion, sodium ion and potassium ion. Of these, the alkali metal ion is preferably a lithium ion from the viewpoint that the viscosity of the ionic liquid can be more easily decreased.

For example, to an aqueous solution of an organic ammonium hydrochloride obtained by dissolving the ammonium hydrochloride in pure water, an aqueous solution of lithium bis(trifluoromethanesulfonyl)imide (Li-TFSI) obtained by dissolving the Li-TFSI in an amount equimolar to that of the ammonium hydrochloride in pure water is gradually added dropwise, and stirred at room temperature for a predetermined period of time (for example, two hours or 24 hours). Thereafter, an extractant is added thereto for liquid separation, and the extraction phase is collected. The collected extraction phase is washed with ultrapure water, followed by vacuum drying, and as a result, it is possible to obtain an ionic liquid of an organic ammonium TFSI which has the same organic moiety as the organic moiety of the organic ammonium hydrochloride as a raw material.

Examples of the extractant include chloroform and toluene. However, chloroform is preferred because chloroform has a high vapor pressure and thus can be easily removed.

In the acid-base neutralization method, the ionic compound can be easily produced by neutralizing an organic amine having an organic moiety having 1 to 20 carbon atoms, with an acid of a fluorine-containing bis(sulfonyl)imide. In other words, the method of producing the ionic compound preferably includes the step of neutralizing such an organic amine, with an acid of a fluorine-containing bis(sulfonyl)imide. The organic amine as used herein is preferably at least one organic amine selected from the group consisting of a primary organic amine, a secondary organic amine, and a tertiary organic amine. Of these, the organic amine is more preferably a primary organic amine. In the case of using the acid-base neutralization method, it is not necessary to use an alkali metal salt of a fluorine-containing bis(sulfonyl)imide as a raw material, and impurities derived from the alkali metal salt are less likely to be incorporated into the hole-transporting layer. Therefore, the use of the acid-base neutralization method is one of the preferred embodiments. The impurities derived from the alkali metal salt as used herein may be, for example, an alkali metal ion.

In the acid-base neutralization method, it is possible to add a solvent to the acid and/or the base to be reacted. It is preferred to add a solvent to the acid and/or the base, because the heat capacity can be increased to reduce the temperature increase of the reaction product of the acid and the base due to the heat generated by the neutralization. In other words, the method of producing the ionic compound preferably include the step of neutralizing the organic amine with an acid of a fluorine-containing bis(sulfonyl)imide in the presence of a solvent.

Further, the solvent is preferably an azeotrope with water, because it is advantageous for removing moisture mixed resulting from the raw materials or from the humidity of the atmosphere. From these points of view, a preferred solvent may be, for example, an alcohol, an ether or the like. More specifically, the solvent is preferably ethanol, toluene, chloroform or chlorobenzene, and most preferably ethanol from the viewpoint of obtaining an excellent affinity with the raw materials and the product. As described above, the method of producing the ionic compound preferably includes the step of removing the solvent.

The primary organic amine is represented by the following Formula (1-1), the secondary organic amine is represented by the following Formula (5-1), and the tertiary organic amine is represented by the following Formula (6-1). Any of the organic moieties described in the section of the organic ammonium cation can be used as the organic moiety.

$$RNH_2 \qquad (1\text{-}1)$$

(In Formula (1-1), R represents an organic moiety having 1 to 20 carbon atoms.)

$$R_2NH \qquad (5\text{-}1)$$

(In Formula (5-1), each R independently represents an organic moiety having 1 to 20 carbon atoms.)

$$R_3N \qquad (6\text{-}1)$$

(In Formula (6-1), each R independently represents an organic moiety having 1 to 20 carbon atoms.)

The acid of the fluorine-containing bis(sulfonyl)imide is represented by the following Formula (2-1).

$$RfSO_2NHSO_2Rf \qquad (2\text{-}1)$$

(In Formula (2-1), each Rf independently represents a fluorine atom, or a fluorine-containing alkyl group having 1 to 10 carbon atoms.)

Examples of the acid of the fluorine-containing bis(sulfonyl)imide include those having a perfluoroalkyl group, such as a protonated product (H-FSI) of bis(fluorosulfonyl)imide, a protonated product (H-TFSI) of bis(trifluoromethanesulfonyl)imide, a protonated product (H-PFSI) of bis(pentafluoroethanesulfonyl)imide, and a protonated product (H-NFSI) of bis(nonafluorobutanesulfonyl)imide.

An example of the acid-base neutralization method is shown below.

For example, to a solution of an organic amine obtained by mixing the organic amine with a solvent such as ethanol, an aqueous solution of a protonated product (H-TFSI) of bis(trifluoromethanesulfonyl)imide obtained by dissolving H-TFSI in an amount equimolar to the organic amine in ethanol is gradually added dropwise, and stirred at room temperature for a predetermined period of time (for example, 30 minutes or two hours) to obtain a product (neutralization step). Thereafter, the resulting product is vacuum-dried at a predetermined temperature (for example, 60°C) for a predetermined period of time (for example, two hours) to remove the solvent. As a result, it is possible to obtain an ionic liquid of an organic ammonium TFSI which has the same organic moiety as the organic moiety of the organic amine as a raw material. The removal of the solvent is preferably performed by placing the product under the condition of reduced pressure (for example, 10 Pa or less) lower than the atmospheric pressure at a temperature of 30°C or higher and 120°C or lower, because the solvent can be removed without decomposing the ionic compound, and efficiently. In other words, the step of removing the solvent preferably includes the step of placing the product produced by the neutralization step under the condition of reduced pressure lower than the atmospheric pressure at a temperature of 30°C or higher and 120°C or lower.

The solvent may be, for example, ethanol, butanol or the like, but it is preferred to use ethanol because the solvent can be removed easily.

The ionic compound according to the present embodiment can be used in various applications, such as electric materials, reagents, solvents for organic synthesis and gas absorbents, because the molecular cation and/or the molecular anion included in the ionic compound has/have an excellent reactivity. As the application of electric material, for example, the ionic compound is suitable for use in solar cell materials, photosensors, luminescent materials, lithium-ion secondary batteries, and the like. The ionic compound is particularly suitable as battery materials, because of its excellent electrical properties. For example, the use of the ionic compound as a dopant for a hole-transporting material in an organic EL panel or a perovskite solar cell facilitates the movement of the holes, making it possible to obtain a high photoelectric conversion efficiency. In particular, the use of the ionic compound as a dopant for a hole-transporting material contained in a hole-transporting layer to be layered on a perovskite layer or the like, imparts hydrophobicity, namely, a high-water resistance, to the perovskite layer, and thus, the ionic compound can be used in a device other than a solar cell that includes a perovskite layer.

(Hole-transporting material)

The "hole-transporting material" as used herein refers, for example, to a semiconductor in which the effective mass of holes is smaller than that of electrons, etc., and is a material which is advantageous in the transportation of holes, or the like. Examples of the hole-transporting material include, for example, a compound that serves as a raw material of an organic substance, an inorganic substance, or the like. However, the hole-transporting material preferably contains an organic substance, because it allows the material to be soft, making it less likely to cause the defects of the resulting film due to bending. Specific examples of the organic substance include assemblies of organic molecules and organic polymers. The organic substance preferably contains an aromatic moiety, from the viewpoint of improving the adhesion force of the hole-transporting layer to the perovskite layer, as described above. Further, as will be described later, the hole-transporting material contains the reaction product of a raw material compound of the hole-transporting material, and the ionic compound according to the present disclosure.

More specific examples of the raw material compound include 2,2',7,7'-tetrakis(N,N-di-p-methoxyphenyl amino)-9,9'-spirobifluorene (Spiro-OMeTAD), poly(3-hexylthiophene-2,5-diyl) (P3HT), poly[bis(4-phenyl)(2,4,6-trimethylphenyl)amine] (PTAA), N,N-bis(3-methylphenyl)-N,N-diphenylbenzidine (TPD), N,N-di[(1-naphthyl)-N,N-diphenyl]-(1,1-biphenyl)-4,4-diamine (NPD), tris(4-carbazolyl-9-ylphenyl)amine (TCTA), poly(9-vinylcarbazole) (PVK) and 4,4-bis(N-carbazolyl)-1,1-biphenyl (CBP), and pyridine compounds such as 4-tert-butylpyridine. One kind or two or more kinds of these compounds may be used. The "pyridine compound" refers to a compound containing a pyridine ring structure within the molecular structure.

In particular, the raw material compound is preferably at least one selected from the group consisting of Spiro-OMeTAD, PTAA, TPD and PVK, and more preferably at least one selected from the group consisting of Spiro-OMeTAD and PTAA, from the viewpoints that: a relatively deep HOMO level and a high carrier density can be achieved, making it advantageous in the transportation of holes; the adhesion force of the hole-transporting layer to the perovskite layer can further be enhanced; and the electromotive force can be increased. Spiro-OMeTAD is more preferred, from the viewpoint of allowing for a particularly effective interaction with the ionic compound, making it possible to improve the performance of the resulting perovskite solar cell. Further, 4-tert-butylpyridine is preferred, from the viewpoint of improving the heat resistance of the resulting solar cell.

The hole-transporting material preferably does not contain an alkali metal ion, from the viewpoint of improving the short circuit current density, the open circuit voltage, the fill factor and the conversion efficiency of the resulting cell. For example, a method using the acid-base neutralization method described above may be used so that the hole-transporting material does not contain an alkali metal ion.

(Use as Dopant for Hole-transporting material)

The hole-transporting material is deposited on a substrate to form a film by a film forming method corresponding to the raw material compound thereof. The substrate can be selected as appropriate depending on the type of the electric material used and the purpose for forming the hole-transporting layer on the substrate. For example, when used as the hole-transporting layer in a perovskite solar cell, the substrate is perovskite or an electrode.

A doping treatment is carried out by mixing the ionic compound and the raw material compound of the hole-transporting material. In the doping treatment described above, the ionic compound and the raw material compound of the hole-transporting material may be mixed in a solvent. For example, the dopant (ionic compound) can be dissolved together, in a solution in which the compound that serves as the raw material of the hole-transporting material has been dissolved. It is also possible to use, not only one kind, but also two or more kinds of the ionic compounds, as the dopant.

By using the ionic compound according to the present embodiment as a dopant for the hole-transporting material, the organic ammonium cation which is derived from the ionic compound and which is a cation having an excellent reactivity, is diffused on the surface of the perovskite layer, and spontaneously reacts with the perovskite layer. This decreases the defects on the perovskite surface and/or improves the electrical bonding interface with the hole-transporting material, thereby improving the solar cell properties such as the open circuit voltage. In addition, it is thought that the reaction of the organic ammonium cation with the surface of the perovskite layer proceeds by the cation portion, such as $NH_3^+$ portion, in the ammonium cation being oriented toward the surface of the perovskite layer. As a result, the organic moiety in the ammonium cation is oriented toward the direction of the surface on the side opposite to the perovskite layer, of the interface between the hole-transporting layer and the perovskite layer, namely oriented on the side of the hole-transporting layer. The above-described orientation of the organic moiety allows the surface of the perovskite layer on the side of the hole-transporting layer to be hydrophobized. When the surface is hydrophobized, solar cell properties with improved water resistance and moisture resistance as well as a long life can be imparted, and thus is preferred.

In other words, when the hole-transporting layer is removed and the water contact angle of the exposed perovskite layer is measured, in a perovskite solar cell including a perovskite layer and a hole-transporting layer formed on the perovskite, the water contact angle is preferably 55.0° or more, more preferably 65.0° or more, and still more preferably 75.0° or more. The upper limit of the water contact angle is not particularly limited. However, the water contact angle may preferably be, for example, from 55.0 to 110°, from 65.0 to 100°, or from 75.0 to 100°. The method of measuring the water contact angle will be described later. The water contact angle can be changed by adjusting the number of carbon atoms in the organic moiety.

Further, as described above, the hole mobility in the hole-transporting material can be improved by the molecular anion including a fluorine-containing bis(sulfonyl)imide. In particular, the combination of the molecular cation and the molecular anion is thought to suitably improve the solar cell performance, by either one or the synergistic effect of both of the followings: namely: the fact that the anion has a high ionicity; and the fact that the negative charge of the anion becomes independent as a result of the reaction between the organic ammonium cation and the perovskite, making it possible to extract electrons more effectively from the hole-transporting material as compared to when the anion coexists with the cation; etc.

Therefore, the hole-transporting material can obtain a high photoelectric conversion efficiency with a high open circuit voltage, making it possible to improve the electrical properties of the hole-transporting material. In particular, the following advantages can be obtained in the case of using the ionic liquid as a dopant.

For example, in the case of using a compound having a high melting point, such as Li-TFSI (melting point: 232°C), as a dopant, there are cases where high temperature conditions are required, or where the dissolution of a solid in a solvent requires time, when preparing a precursor solution of the hole-transporting material (film). Further, there are also cases where it is difficult to perform an accurate weighing due to factors such as static electricity, at the time of weighing a solid.

In contrast, since the ionic liquid has a low melting point, the precursor solution can be prepared only by mixing with the solvent, at normal temperature or under mild heating conditions of equal to or lower than the boiling point of the solvent. Therefore, the preparation step of the precursor solution can be simplified, and thus is suitable. For example, an ionic liquid having a melting point of less than 35°C, preferably 25°C or lower, is more preferred, because the ionic liquid is liquid even at normal temperature, and thus the preparation of the precursor solution can be performed under mild conditions. Such an ionic liquid may be, for example, an ionic liquid including: an organic ammonium cation containing an alkyl group having, for example, from 2 to 12 carbon atoms, from 4 to 20 carbon atoms, or from 4 to 12 carbon atoms; and TFSI. Specific examples of such an ionic liquid include n-butylammonium TFSI (melting point: 23°C), n-octylammonium TFSI (melting point: less than -70°C), n-dodecane ammonium TFSI (melting point: less than 10°C), n-octylammonium FSI (melting point: less than 0°C), n-methyl-n-octylammonium TFSI (melting point: 1°C), n,n-dimethyl-n-octylammonium TFSI (melting point: -35°C), n-octylammonium FSI (melting point: -24°C) and n-octylammonium PFSI (melting point: 7°C).

(Perovskite Solar Cell)

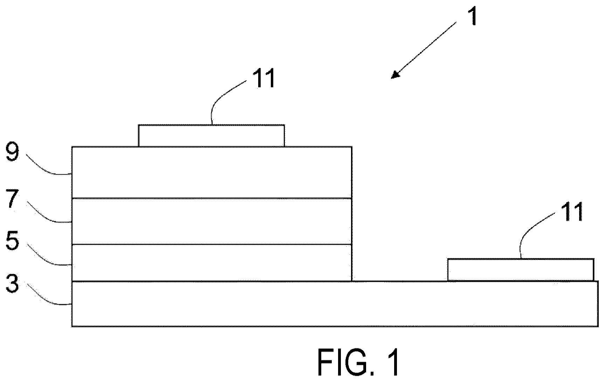

The hole-transporting material prepared by the method described above and subjected to the doping treatment can be used as a material of a hole-transporting layer in an organic EL panel, a perovskite solar cell, or the like. An example of the case in which the hole-transporting material is used as a material of a hole-transporting layer in a perovskite solar cell, for example, is shown below. FIG. 1 is a schematic diagram showing one example of the basic configuration of a perovskite solar cell 1.

The perovskite solar cell 1 is produced by forming an electron-transporting layer 5, a perovskite layer 7 and a hole-transporting layer 9, between an electrically conductive substrate 3, and an electrode 11 which is an electrode (cathode). Further, another electrode (anode) 11 is formed on the electrically conductive substrate 3. In other words, the perovskite solar cell 1 preferably includes the perovskite layer 7, and the hole-transporting layer 9 formed on the perovskite layer 7, and more preferably includes: the electrically conductive substrate 3, the electron-transporting layer 5 and the electrode 11 formed on the electrically conductive substrate 3, the perovskite layer 7 formed on the electron-transporting layer 5, the hole-transporting layer 9 formed on the perovskite layer 7, and the electrode 11 formed on the hole-transporting layer.

In the perovskite solar cell 1, incident light from below is absorbed by the perovskite layer 7, to generate electrons and holes. The electrons and the holes move to the electron-transporting layer 5 and the hole-transporting layer 9, respectively, and the photoelectromotive force is generated between both electrodes, to generate a current. The perovskite solar cell 1 can have a structure in which the electrically conductive substrate 3 is transparent. In this case, light can be irradiated from above to generate power.

(Electrically Conductive Substrate)

The electrically conductive substrate 3 is a substrate made of an electrically conductive material. Examples of the electrically conductive material include: metals such as platinum and gold; carbon; and electrically conductive metal oxides such as fluorine-doped tin oxide (FTO) and indium tin oxide (ITO). Of these, FTO and ITO are suitable, because these oxides are transparent. While not shown in the figure, a transparent support substrate such as a glass substrate or a plastic substrate may be provided under the electrically conductive substrate 3.

The electrically conductive substrate 3 preferably has a thickness of from about 100 nm to 1,000 nm, but is not particularly limited thereto. The electrically conductive substrate 3 can be obtained, for example, by molding the electrically conductive material in the form of a flat plate. When the perovskite solar cell 1 includes a support substrate, the electrically conductive substrate 3 can be obtained by layering the electrically conductive material on the support substrate.

(Electron-transporting layer)

The electron-transporting layer 5 is formed on the electrically conductive substrate 3. The electron-transporting layer 5 contains an electron-transporting material. The electron-transporting material in the electron-transporting layer 5 may contain, for example, an organic substance or an inorganic substance. However, the electron-transporting material preferably contains an inorganic substance because a high strength can be obtained. The inorganic substance is more preferably a metal compound, because the physical properties can be adjusted relatively easily. The inorganic substance is still more preferably a metal oxide, because the metal oxide can be produced and stored relatively easily in the air atmosphere. In other words, the electron-transporting layer is preferably a metal oxide layer.

Specific examples of the metal oxide include, but not limited to, titanium oxide ($TiO_2$), zinc oxide (ZnO), niobium oxides (such as $Nb_2O_5$), tungsten oxides (such as $WO_2$, $WO_3$ and $W_2O_3$), tin oxide ($SnO_2$), aluminum oxide ($Al_2O_3$), silicon oxide ($SiO_2$), magnesium oxide (MgO), and zirconium oxide ($ZrO_2$). Of these, titanium oxide and niobium oxides are preferred, from the viewpoint that the effective mass of electrons is small. Above all, titanium oxide is preferred, from the viewpoint that the material thereof is abundant and inexpensive.

The thickness of the electron-transporting layer 5 is preferably from about 10 to 1,000 nm, for example, from the viewpoint that more electrons can be collected from the perovskite layer 7 formed thereon. The electron-transporting layer 5 can be obtained using a known film forming method, depending on the material to be molded. In the case of using tin oxide, for example, the electron-transporting layer 5 can be formed by coating a colloidal solution of tin oxide on the electrically conductive substrate 3, followed by heating (for example, at a temperature of from 100 to 150°C) and drying. In the case of using titanium oxide, the electron-transporting layer 5 can be formed by coating an alcohol solution (such as ethanol solution or an isopropanol solution) of a titanium oxide paste. In the case of using aluminum oxide, zirconium oxide, niobium oxide or the like, the electron-transporting layer 5 can be formed in the same manner, by preparing a paste of each oxide and performing the coating. A known or a commercially available product can be used as the colloidal solution of tin oxide or the titanium oxide paste. A treatment in which a precursor material of the electron-transporting material is adsorbed on, or allowed to react with, the electron-transporting material may be carried out, from the viewpoint of decreasing the defects of the electron-transporting material, such as pinholes. Specifically, in the case of using titanium oxide, it is preferred to carry out a treatment ($TiCl_4$ treatment) in which a titanium chloride species is adsorbed on the electron-transporting material such as titanium oxide, followed by hydrolysis to allow titanium oxide to bonded thereto.

(Perovskite Layer)

The perovskite layer 7 that functions as a photoelectric conversion layer is formed on the electron-transporting layer 5. In the perovskite layer 7, electrons and holes are generated by light irradiation. The perovskite layer contains a perovskite material represented by General Formula $ABX_3$ (wherein A represents an organic molecular cation (in a form containing $NH_3$ or the like), or potassium, cesium or rubidium; B represents a metal atom; and X represents a halogen atom), or a perovskite complex thereof. Examples of the perovskite material or the perovskite complex include $APbX_3$ and $ASnX_3$. More specific examples thereof include: $CH_3NH_3PbI_3$, $(CHN_2H_4)PbI_3$, $CsPbX_3$, and $RbPbX_3$; and those in which a plurality of kinds of these As are complexed.

The perovskite layer 7 preferably has a film thickness of, for example, from 100 to 600 nm, from the viewpoints of the balance between light absorption efficiency and exciton diffusion length, and the absorption efficiency of the light transmitted through the electrically conductive substrate 3. The perovskite layer 7 can be formed by dissolving components that form the perovskite layer 7 in a solvent, and coating the resulting solution on the electron-transporting layer 5, followed by drying. Examples of the solvent which can be preferably used include: esters such as γ-butyrolactone, methyl formate and ethyl acetate; ketones such as acetone and dimethyl ketones; ethers such as diethyl ether, diisopropyl ether; alcohols such as methanol and ethanol; halogenated hydrocarbons such as ethylene chloride and chloroform; nitrile solvents such as acetonitrile and propionitrile; N,N-dimethylformamide; and dimethyl sulfoxide. Of these, at least one selected from the group consisting of N,N-dimethylformamide and dimethyl sulfoxide is preferred. The perovskite layer 7 may be formed by coating a precursor of the perovskite layer 7, and then applying a poor solvent such as toluene thereon.

(Hole-transporting layer)

The hole-transporting layer 9 is formed on the perovskite layer 7. The hole-transporting material described above can be used for the hole-transporting layer 9. In other words, the hole-transporting layer 9 preferably contains the hole-transporting material according to the present disclosure. The hole-transporting layer 9 can be formed by coating a solution in which the materials of the hole-transporting layer 9 such as the hole-transporting material and the like are dissolved, on the perovskite layer 7, followed by drying. The hole-transporting layer 9 preferably has a thickness of from about 10 to 1,000 nm. The thickness of the hole-transporting layer 9 is more preferably from about 50 to 500 nm, because a thickness sufficient for preventing the formation of pinholes is required, and such a thickness allows for achieving a low resistance. The amount of the dopant (ionic compound) is desirably set to such an amount that allows the hole-transporting material to be sufficiently oxidized while preventing overoxidation. For example, the amount of the dopant is preferably 3 mM or more and 80 mM or less with respect to the total amount of the solution, from the viewpoint described above. Further, the amount of the dopant is preferably 4 % by mole or more and 111 % by mole or less with respect to the amount of the hole-transporting material, from the viewpoint described above.

The average adhesion force of the hole-transporting layer to the perovskite layer is preferably $1.0\,N\cdot cm^{-1}$ or more. When the average adhesion force is within the above-described range, the adhesion between the hole-transporting layer and the perovskite layer can be more easily improved, making it easier to improve the durability of the resulting perovskite solar cell. The upper limit of the average adhesion force may be, for example, $1.0\,N\cdot cm^{-1}$ or more and $10.0\,N\cdot cm^{-1}$ or less, $1.0\,N\cdot cm^{-1}$ or more and $5.0\,N\cdot cm^{-1}$ or less, or $1.0\,N\cdot cm^{-1}$ or more and $2.0\,N\cdot cm^{-1}$ or less, but not particularly limited thereto. As described above, the average adhesion force can be adjusted, for example, by changing the structure of the organic ammonium, or changing the materials contained in the hole-transporting layer. The method of measuring the average adhesion force will be described later, but the measurement is performed using a TENSILON Type UCT-5T (manufactured by Orientec Co., Ltd.) and a Kapton tape, at a crosshead speed of 100 mm/min. The average adhesion force is defined as the arithmetic mean value of the measured values of the adhesion force within the range of crosshead length from 10 to 50 mm, per tape width.

There are cases where it is necessary to perform a treatment step (for example, the step of coating a solution in which n-octylammonium iodide salt is dissolved, followed by drying, or the like) on the interface between the hole-transporting layer 9 and the perovskite layer 7, in order to stabilize the surface of the perovskite layer 7. However, the present embodiment is significantly and industrially advantageous in that the state of the interface between the perovskite and the hole-transporting material can be improved without requiring such a treatment step. Therefore, a perovskite solar cell can be produced by a simple method including a smaller number of steps. In addition, the organic ammonium cation in the hole-transporting layer reacts with the surface of the perovskite layer, as described above. In other words, the hole-transporting layer preferably contains the reaction product of the organic ammonium cation and the perovskite layer.

(Electrodes)

Finally, the solar cell 1 (solar cell element) can be produced by forming the electrode 11 on each of the electrically conductive substrate 3 which is transparent, and on the hole-transporting layer 9. Each electrode 11 is formed, for example, of gold (Au). A metallic material such as silver, copper, aluminum or nickel may also be used. Each electrode 11 can be formed by any of the known methods such as vapor deposition, sputtering, spraying, spin coating and dip coating.

(Method of Producing Perovskite Solar Cell)

As described above, the method of producing a perovskite solar cell according to the present disclosure is a method of producing a perovskite solar cell by layering an electron-transporting layer, a perovskite layer and a hole-transporting layer between an electrically conductive substrate and an electrode. The method of producing a perovskite solar cell includes the step of forming the hole-transporting layer. Further, the step of forming the hole-transporting layer includes the step of forming a film on the perovskite layer, using the hole-transporting material according to the present disclosure.

In addition, the method of producing a perovskite solar cell preferably includes: the step of preparing the electrically conductive substrate; the step of forming the electron-transporting layer on the electrically conductive substrate; the step of forming the perovskite layer on the electron-transporting layer; the step of forming the hole-transporting layer on the perovskite layer; and the step of forming the electrode on the electrically conductive substrate and on the hole-transporting layer.

EXAMPLES

The present embodiment will be described below more specifically, with reference to specific Examples and Comparative Examples. However, the present embodiment is not limited in any way to these Examples and Comparative Examples, as long as the gist of the present embodiment is not exceeded. The physical properties, reaction conditions and product identification in the Examples and Comparative Examples to be described later are measured and set by the methods shown below.

(Evaluation of Solar Cell Properties)

The solar cell properties of each of the cells produced by the following method were evaluated using the following apparatuses, and by the following evaluation methods.

[Apparatuses]

(Simulated Sunlight)

A simulated sunlight was generated using a solar simulator (manufactured by Seric Ltd.), with the irradiation dose adjusted to 100 mW/cm$^2$ using a photodiode. The irradiation area of the simulated sunlight to the irradiation cell was 0.119 cm$^2$ in any of the cells.

(Direct Current Power Supply)

The application of a voltage to each cell, the evaluation of the photocurrent value, and the operation of sweeping the applied voltage were carried out using a DC voltage/current generator (product number: R6243, manufactured by Advantest Corporation), and the voltage was swept from the high potential side to the low potential side, or from the low potential side to the high potential side.

[Evaluation Methods]

(Short Circuit Current Density)

The short-circuit current was determined from the density of the photocurrent flowed when the simulated sunlight described above was irradiated to each produced cell without applying a voltage thereto.

(Open Circuit Voltage)

The open circuit voltage was determined from the applied voltage to each cell at which the current value reaches 0, when the simulated sunlight described above was irradiated to the cell.

(Fill Factor)

The fill factor (hereinafter, also referred to as "F. F.") was determined by: obtaining the value (actual maximum power value) at which the power reaches its maximum, when the applied voltage was swept while irradiating the simulated sunlight described above; and dividing the thus obtained value by the product of the short circuit current density and the open circuit voltage, in accordance with the following Formula (3).

(F. F.) = (actual maximum power value)/((short circuit current density) × (open circuit voltage))     (3)

(Solar Energy Conversion Efficiency)

The solar energy conversion efficiency (hereinafter, also referred to as "conversion efficiency") was determined in accordance with the following Formula (4).

$$(\text{Conversion efficiency}) = (\text{actual maximum power value } (\text{mW/cm}^{-2}))/100 \text{ mW/cm}^{-2}$$

$$(4)$$

[Water Contact Angle]

The water contact angle was determined using a contact angle meter (product number: DMo-401, manufactured by Kyowa Interface Science Co., Ltd.), by dropping 1 mL of distilled water on a sample, and measuring the contact angle between the sample of 2 seconds after the dropping and water droplets. The method of preparing the sample will be described later.

(Example 1)

(Production of Perovskite Solar Cell)

A glass substrate (manufactured by Nippon Sheet Glass Company, Ltd.) on which FTO (fluorine-doped tin oxide) had been deposited was used as an electrically conductive substrate, and a 50-nm $TiO_2$ dense layer as an electron-transporting material was formed on the FTO surface. Each of the FTO and the dense layer was formed by spray pyrolysis. On the thus formed dense layer, a layer of $TiO_2$ particles having a particle size of 15 nm was deposited to a film thickness of 200 nm by spin coating, to obtain an electron-transporting layer. On top of the electron-transporting layer, a solution obtained by dissolving a single crystal of 1.8 mM of formamidinium lead iodide ($FAPbI_3$; synthesized by recrystallization from FAI and $PbI_2$ manufactured by Tokyo Chemical Industry Co., Ltd.) in a mixture of N,N-dimethylformamide (manufactured by Sigma-Aldrich Co. LLC) and dimethyl sulfoxide (manufactured by Sigma-Aldrich Co. LLC) mixed at a volume ratio of 4:1 was spin-coated at 6,000 rpm for 50 seconds, and 1 mL of chlorobenzene (manufactured by Sigma-Aldrich Co. LLC) was dropped thereon 10 seconds after the start of the spin coating. After the spin coating described above, the resulting sample was heated at 100°C for 10 minutes, to form a thin perovskite layer. On the thus formed thin film, a Spiro-OMeTAD-based hole-transporting layer precursor solution which contains an ionic compound prepared under the following conditions as a dopant, was spin-coated at 3,000 rpm for 30 seconds to layer the hole-transporting material, thereby forming a hole-transporting layer. The method of preparing the hole-transporting layer precursor solution will be described later. Finally, gold (manufactured by The Nilaco Corporation) was deposited by vacuum vapor deposition on each of the FTO and the hole-transporting layer to a thickness of 200 nm, to prepare electrodes.

The ionic compound as a dopant was prepared by the following method (ion exchange method).

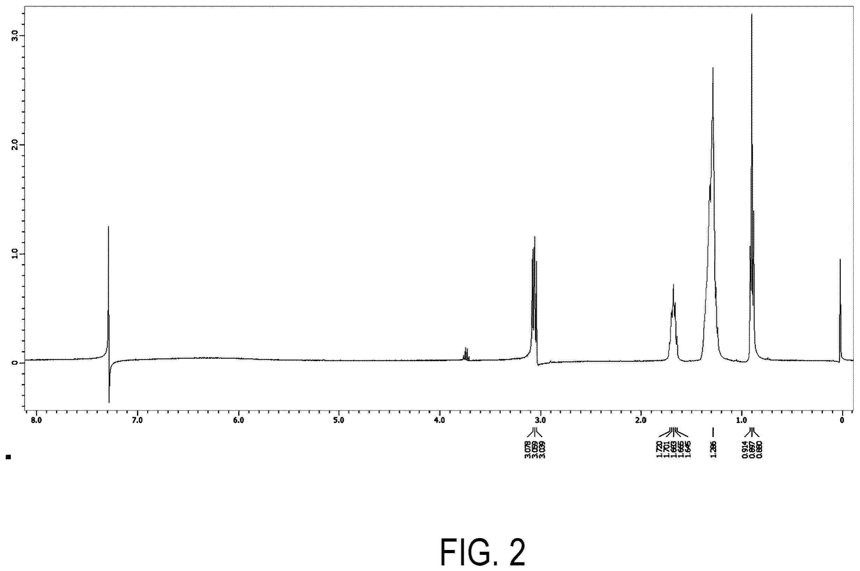

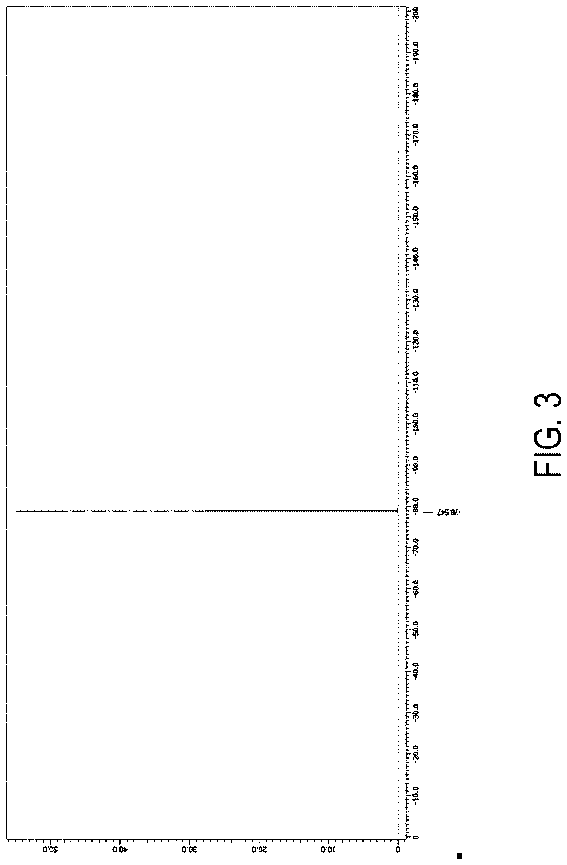

Under a nitrogen gas stream, 5.23 g (32 mmol) of octylamine hydrochloride (manufactured by Tokyo Chemical Industry Co., Ltd.) was weighed into a 200 mL two-necked flask, and dissolved in 50 mL of pure water. To the resulting aqueous solution, 50 mL of an aqueous solution of 9.06 g (32 mmol) of lithium bis(trifluoromethanesulfonyl)imide (manufactured by Tokyo Chemical Industry Co., Ltd.) was added, followed by stirring at room temperature for 24 hours. Twenty-four hours later, a chloroform solution (manufactured by Kishida Chemical Co., Ltd.) was added for liquid separation, and the chloroform layer was washed twice with ultrapure water. Thereafter, chloroform was removed from the chloroform solution at 40°C using an evaporator, followed by vacuum drying at room temperature, to obtain an ionic liquid of n-octylammonium TFSI. The identification of n-octylammonium TFSI was carried out by $^1$H-NMR (FIG. 2) and $^{19}$F-NMR (FIG. 3) using deuterated chloroform.

$^1$H-NMR and $^{19}$F-NMR were measured using a nuclear magnetic resonance spectrometer (product number: AVANCE 400, manufactured by Bruker Corporation). A quantity of 5 mg of a sample of the ionic liquid was mixed with 1 mL of deuterated chloroform to prepare a measurement sample.

The melting point was confirmed to be less than -70°C, because no change in the quantity of heat attributed to the solidification point was observed when the temperature was swept from 25°C to -70°C using a differential scanning calorimeter (product number: DSC7020, manufactured by Hitachi High-Tech Science Corporation).

The amount of lithium contained in the ionic liquid was evaluated using a high frequency inductively coupled plasma emission spectrometer (ICP-OES710, manufactured by Agilent Technologies, Inc.). As a result, the amount of lithium was 2.8 ppm by mass.

**EP 4 660 180 A1**

The method of preparing the hole-transporting layer precursor solution was as follows.

A solution obtained by dissolving 70 mM of Spiro-OMeTAD (manufactured by Nippon Fine Chemical Co., Ltd.), 12 mM of the n-octylammonium TFSI obtained as described above, and 34 mM of t-butylpyridine (manufactured by Tokyo Chemical Industry Co., Ltd.) in chlorobenzene, was used as the hole-transporting layer precursor solution.

The solar cell properties were evaluated by irradiating a simulated sunlight to the cell obtained as described above, from the side of the FTO glass. The evaluation results are shown in Table 1. For the evaluation, 15 perovskite solar cells were produced by the same process, and Table 1 shows the arithmetic mean value of the 15 cells, for each solar cell property. Table 1 shows the arithmetic mean value of the 15 cells, along with the error calculated from the standard deviation thereof. In each of the following Examples and Comparative Examples, the cell production and evaluations were carried out by the same methods and under the same conditions as in Example 1, and the same reagent was used for the common compound, unless otherwise defined. The apparatuses and conditions used for the measurements were also the same as those in Example 1, unless otherwise defined.

(Example 2)

A perovskite solar cell was produced in the same manner and under the same conditions as in Example 1, except that: $TiO_2$ particles having an average particle size of 30 nm were used for the $TiO_2$ particle layer; the ionic compound was prepared by the following method; and a solution obtained by dissolving 70 mM of Spiro-OMeTAD (manufactured by Nippon Fine Chemical Co., Ltd.), 24 mM of n-octylammonium TFSI prepared by the following method and 102 mM of the t-butylpyridine in chlorobenzene, was used as the hole-transporting layer precursor solution.

The ionic compound as a dopant was prepared by the following method (acid-base neutralization method).

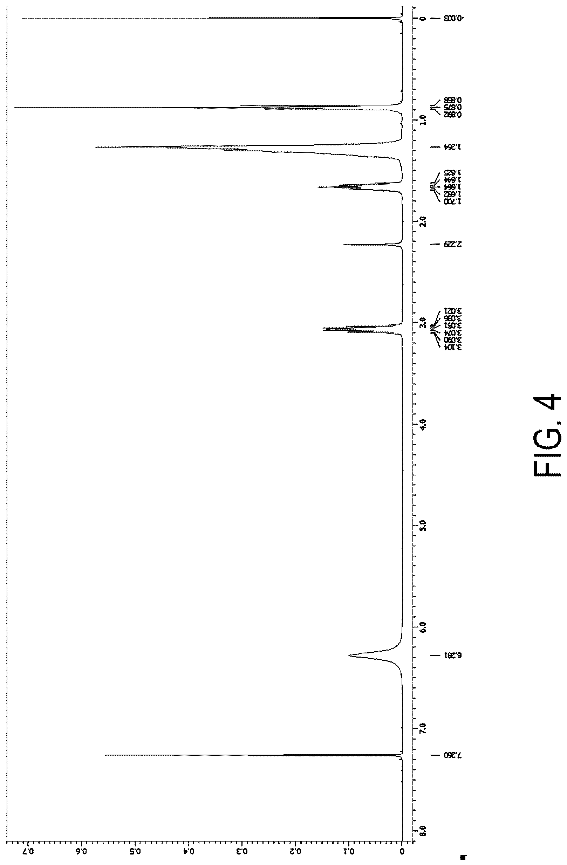

Ethanol (93 mL) was introduced into a 500 mL four-neck flask, and the flask was immersed in an ice bath. To the flask, H-TFSI (13.06 g, 46.4 mmol, manufactured by Tokyo Chemical Industry Co., Ltd.) was added to prepare a homogeneous solution. Thereafter, octylamine (6.03 g, 46.6 mmol, manufactured by Tokyo Chemical Industry Co., Ltd.) in ethanol (93 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at temperatures of 40°C, 50°C and 60°C, for two hours at each temperature, to obtain an ionic compound (n-octylammonium TFSI) in the form of a liquid. The results of the $^1$H-NMR measurement of the resulting ionic compound are shown in FIG. 4. $^1$H-NMR was measured using a nuclear magnetic resonance spectrometer (product number: JNM-ECZ400S/L1, manufactured by JEOL Ltd.), and using a solution obtained by mixing the resulting ionic compound with a deuterated chloroform solvent as the measurement sample. In each of the following Examples 3 to 6, $^1$H-NMR was measured using the same apparatus as that used in Example 2 and under the same conditions.

(Example 3)

A perovskite solar cell was produced in the same manner and under the same conditions as in Example 2, except that: the ionic compound was prepared by the following method; and a solution obtained by dissolving 70 mM of Spiro-OMeTAD (manufactured by Nippon Fine Chemical Co., Ltd.), 48 mM of n-dodecylammonium TFSI prepared by the following method and 204 mM of the t-butylpyridine in chlorobenzene, was used as the hole-transporting layer precursor solution.

The ionic compound as a dopant was prepared by the following method (acid-base neutralization method).

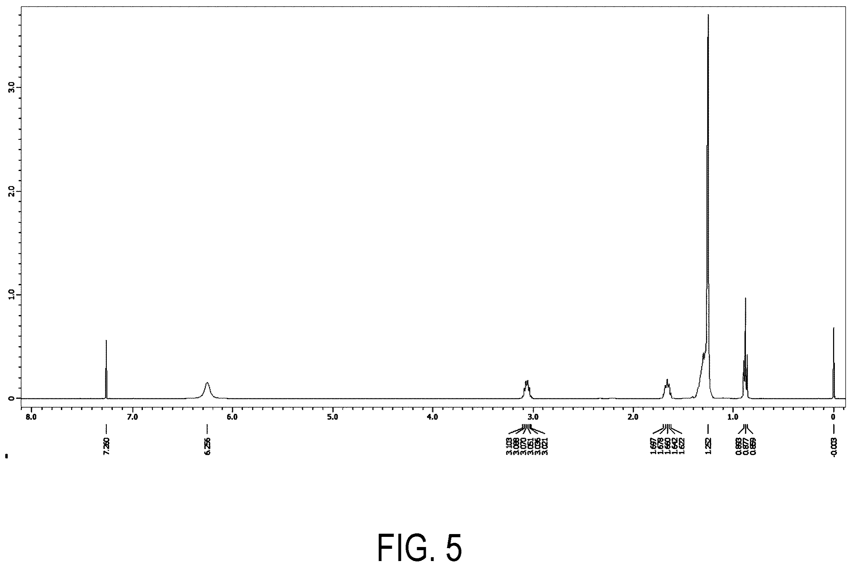

Ethanol (75 mL) was introduced into a 500 mL four-neck flask, and the flask was immersed in an ice bath. To the flask, H-TFSI (10.61 g, 37.7 mmol, manufactured by Tokyo Chemical Industry Co., Ltd.) was added to prepare a homogeneous solution. Thereafter, dodecylamine (7.02 g, 37.7 mmol, manufactured by Tokyo Chemical Industry Co., Ltd.) in ethanol (75 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at temperatures of 40°C, 60°C and 80°C, for two hours at each temperature, to obtain an ionic compound (n-dodecylammonium TFSI) in the form of a liquid. The results of the $^1$H-NMR measurement of the resulting ionic compound are shown in FIG. 5.

(Example 4)

A perovskite solar cell was produced in the same manner and under the same conditions as in Example 3, except that: the ionic compound was prepared by the following method; and a solution obtained by dissolving 70 mM of Spiro-OMeTAD (manufactured by Nippon Fine Chemical Co., Ltd.), 18 mM of n-butylammonium TFSI prepared by the following

**17**

method and 77 mM of the t-butylpyridine in chlorobenzene, was used as the hole-transporting layer precursor solution.

The ionic compound as a dopant was prepared by the following method (acid-base neutralization method).

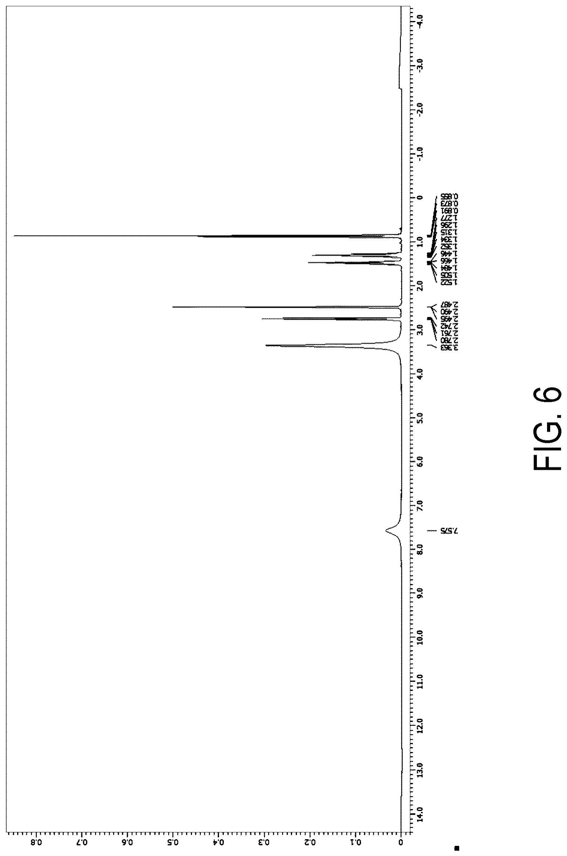

Ethanol (110 mL) was introduced into a 500 mL four-neck flask, and the flask was immersed in an ice bath. To the flask, H-TFSI (15.38 g, 54.7 mmol, manufactured by Tokyo Chemical Industry Co., Ltd.) was added to prepare a homogeneous solution. Thereafter, butylamine (4.03 g, 54.7 mmol, manufactured by Tokyo Chemical Industry Co., Ltd.) in ethanol (110 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at temperatures of 40°C, 60°C and 80°C, for two hours at each temperature, to obtain an ionic compound (n-butylammonium TFSI) in the form of a liquid. The results of the $^1$H-NMR measurement of the resulting ionic compound are shown in FIG. 6.

(Example 5)

A perovskite solar cell was produced in the same manner and under the same conditions as in Example 2, except that: the ionic compound was prepared by the following method; and a solution obtained by dissolving 70 mM of Spiro-OMeTAD (manufactured by Nippon Fine Chemical Co., Ltd.), 18 mM of n- ethylammonium TFSI prepared by the following method and 77 mM of the t-butylpyridine in chlorobenzene, was used as the hole-transporting layer precursor solution.

The ionic compound as a dopant was prepared by the following method (acid-base neutralization method).

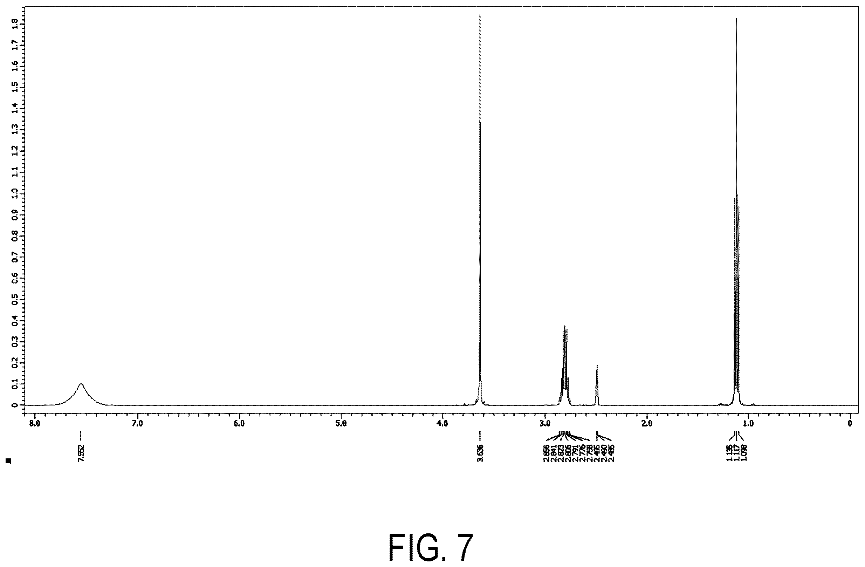

Ethanol (102 mL) was introduced into a 500 mL four-neck flask, and the flask was immersed in an ice bath. To the flask, H-TFSI (14.37 g, 51.1 mmol, manufactured by Tokyo Chemical Industry Co., Ltd.) was added to prepare a homogeneous solution. Thereafter, ethylamine (6.72g, 51.1 mmol, manufactured by Tokyo Chemical Industry Co., Ltd., 34.3% by mass ethylamine/ethanol solution in ethanol (96 mL), ethylamine) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at temperatures of 40°C, 60°C and 80°C, for two hours at each temperature, to obtain an ionic compound (ethylammonium TFSI) in the form of a solid. The results of the $^1$H-NMR measurement of the resulting ionic compound are shown in FIG. 7. Further, the melting point, as determined from the result of the measurement using the differential scanning calorimeter, was 45°C.

(Example 6)

A perovskite solar cell was produced in the same manner and under the same conditions as in Example 2, except that: the ionic compound was prepared by the following method; and a solution obtained by dissolving 70 mM of Spiro-OMeTAD (manufactured by Nippon Fine Chemical Co., Ltd.), 48 mM of 2-phenylethylammonium TFSI prepared by the following method and 204 mM of the t-butylpyridine in chlorobenzene, was used as the hole-transporting layer precursor solution.

The ionic compound as a dopant was prepared by the following method (acid-base neutralization method).

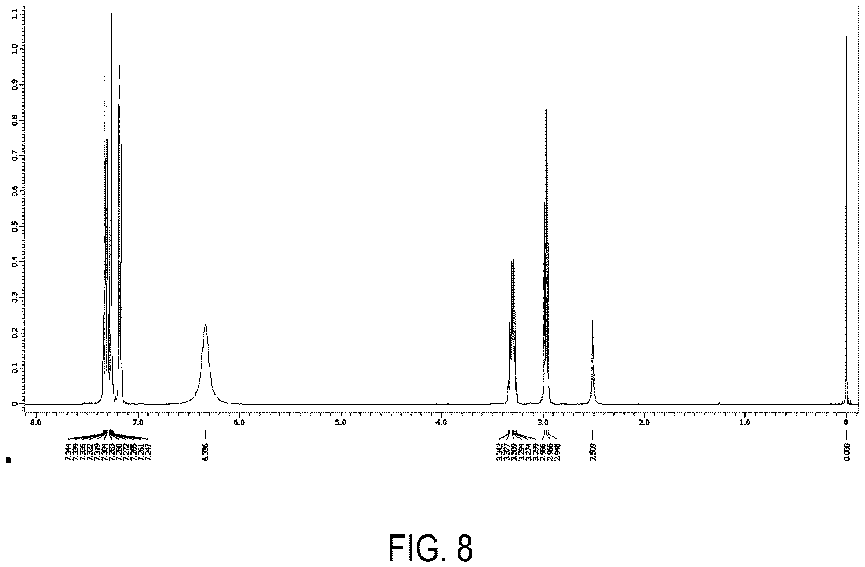

Ethanol (83 mL) was introduced into a 500 mL four-neck flask, and the flask was immersed in an ice bath. To the flask, H-TFSI (11.6 g, 41.4 mmol, manufactured by Tokyo Chemical Industry Co., Ltd.) was added to prepare a homogeneous solution. Thereafter, 2-phenylethylamine (5.02 g, 41.4 mmol) in ethanol (83 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at temperatures of 40°C, 60°C and 80°C, for two hours at each temperature, to obtain an ionic compound (2-phenylethylammonium TFSI) in the form of a solid. The results of the $^1$H-NMR measurement of the resulting ionic compound are shown in FIG. 8. The melting point, as determined from the result of the measurement using the differential scanning calorimeter, was 35°C.

(Example 7)

A perovskite solar cell was produced in the same manner and under the same conditions as in Example 2, except that: the ionic compound was prepared by the following method; and a solution obtained by dissolving 70 mM of Spiro-OMeTAD (manufactured by Nippon Fine Chemical Co., Ltd.), 48 mM of n-methyl-n-octylammonium TFSI prepared by the following method and 204 mM of the t-butylpyridine in chlorobenzene, was used as the hole-transporting layer precursor solution.

The ionic compound as a dopant was prepared by the following method (acid-base neutralization method).

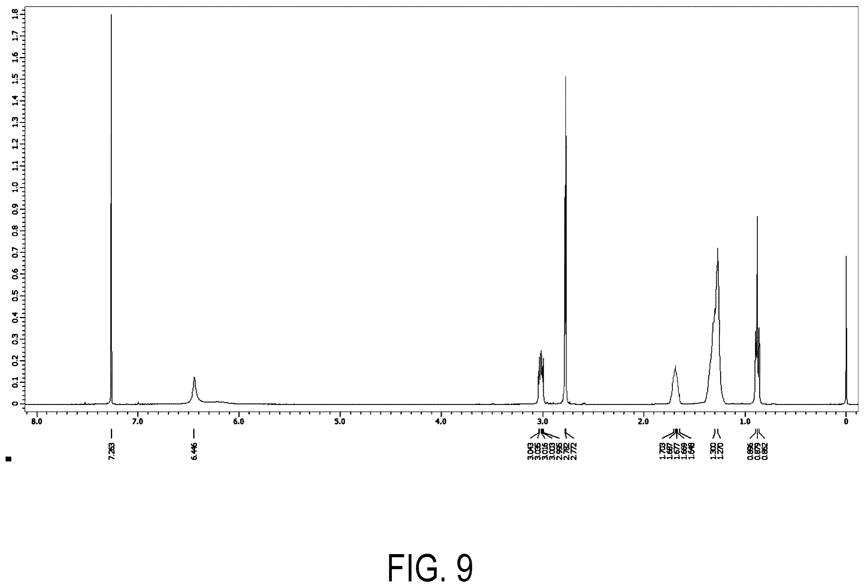

Ethanol (83 mL) was introduced into a 500 mL four-neck flask, and the flask was immersed in an ice bath. To the flask, H-TFSI (15.79 g, 56.2 mmol, manufactured by Tokyo Chemical Industry Co., Ltd.) was added to prepare a homogeneous solution. Thereafter, n-methyl-n-octylamine (8.05 g, 56.2 mmol) in ethanol (100 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at 60°C for 12 hours, to obtain an ionic compound (n-methyl-n-octylammonium TFSI) in the form of a solid. The results of the $^1$H-NMR measurement of the resulting ionic compound are shown in FIG. 9. The melting point, as determined from the result of the measurement using the differential scanning calorimeter, was 1°C.

(Example 8)

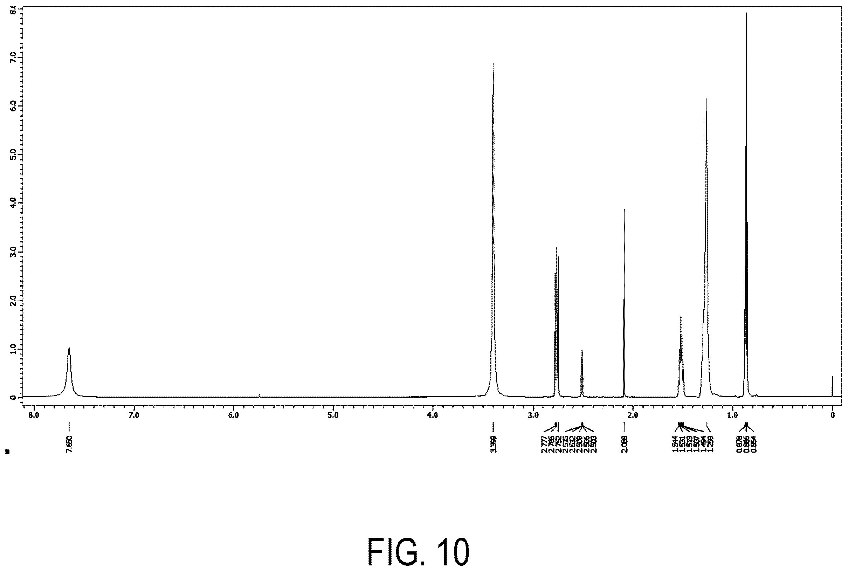

A perovskite solar cell was produced in the same manner and under the same conditions as in Example 2, except that: the ionic compound was prepared by the following method; and a solution obtained by dissolving 70 mM of Spiro-OMeTAD (manufactured by Nippon Fine Chemical Co., Ltd.), 24 mM of n-octylammonium FSI prepared by the following method and 204 mM of the t-butylpyridine in chlorobenzene, was used as the hole-transporting layer precursor solution.
The ionic compound as a dopant was prepared by the following method (ion exchange method).
Under a nitrogen gas stream, 6.63 g (40 mmol) of octylamine hydrochloride (manufactured by Tokyo Chemical Industry Co., Ltd.) was weighed into a 200 mL two-necked flask, and dissolved in 20 mL of pure water. To the resulting aqueous solution, 20 mL of an aqueous solution of 7.48 g (40 mmol) of lithium bis(fluorosulfonyl)imide (manufactured by Tokyo Chemical Industry Co., Ltd.) was added, followed by stirring at room temperature for two hours. Two hours later, a chloroform solution (manufactured by Kishida Chemical Co., Ltd.) was added for liquid separation, and the chloroform layer was washed twice with ultrapure water. Thereafter, chloroform was removed from the chloroform solution at 40°C using an evaporator, followed by vacuum drying at room temperature, to obtain an ionic liquid of n-octylammonium FSI. The results of the $^1$H-NMR measurement of the resulting ionic compound are shown in FIG. 10. The melting point, as determined from the result of the measurement using the differential scanning calorimeter, was -24°C. Further, the lithium content was 6.7 ppm by mass.

(Comparative Example 1)

A perovskite solar cell was produced and evaluated in the same manner as in Example 1, except that a solution obtained by dissolving 70 mM of Spiro-OMeTAD in chlorobenzene was used as the hole-transporting material precursor solution.

(Comparative Example 2)

A perovskite solar cell was produced and evaluated in the same manner as in Example 1, except that a solution obtained by dissolving 70 mM of Spiro-OMeTAD, 48 mM of Li-TFSI (manufactured by Sigma-Aldrich Co. LLC) and 272 mM of the t-butylpyridine in chlorobenzene was used as the hole-transporting material precursor solution.

(Comparative Example 3)

A perovskite solar cell was produced and evaluated in the same manner as in Example 1, except that a solution obtained by dissolving 70 mM of Spiro-OMeTAD, 34 mM of methyltri-n-octylammonium TFSI and 10 mM of the t-butylpyridine in chlorobenzene was used as the hole-transporting material precursor solution.

(Comparative Example 4)

A perovskite solar cell was produced and evaluated in the same manner as in Example 1, except that a solution obtained by dissolving 70 mM of Spiro-OMeTAD, 24 mM of 1-ethyl-3-methylimidazolium TFSI and 64 mM of the t-butylpyridine in chlorobenzene was used as the hole-transporting material precursor solution.
The evaluation results of these Examples and Comparative Examples are shown in Table 1 and Table 2.

[Table 1]

Table 1

| | dopant material | degree of substitution of ammonium | synthesis method | forward scan | | | |
|---|---|---|---|---|---|---|---|
| | | | | short circuit current density Jsc (mA/cm$^2$) | open circuit voltage Voc (V) | fill factor FF | conversion efficiency $\eta$ (%) |
| Example 1 | n-octylammonium TFSI | primary | ion exhange | 23.46±0.55 | 1.02±0.02 | 0.70±0.01 | 16.86±0.59 |
| Example 2 | n-octylammonium TFSI | primary | neutralization | 26.45±0.14 | 1.13±0.02 | 0.74±0.01 | 22.11±0.58 |
| Example 3 | n-dodecylammonium TFSI | primary | neutralization | 26.07±0.90 | 1.00±0.02 | 0.63±0.04 | 16.38±1.47 |
| Example 4 | n-butylammonium TFSI | primary | neutralization | 26.25±0.43 | 1.01±0.04 | 0.68±0.01 | 18.11±0.69 |
| Example 5 | ethylammonium TFSI | primary | neutralization | 26.00±0.15 | 1.01±0.04 | 0.68±0.01 | 18.82±1.57 |
| Example 6 | 2-phenylethylammonium TFSI | primary | neutralization | 26.54±0.16 | 1.02±0.01 | 0.53±0.02 | 14.30±0.45 |
| Example 7 | N-methyl-n-octylammonium TFSI | secondary | neutralization | 25.32±0.25 | 0.97±0.02 | 0.54±0.02 | 13.13±0.87 |
| Example 8 | n-octylammonnium FSI | primary | ion exchange | 25.43 ± 0.16 | 0.92±0.02 | 0.63±0.02 | 14.65±0.75 |
| Comparative Example 1 | none | none | - | 10.65±3.52 | 0.72±0.22 | 0.25±0.02 | 1.98±0.92 |
| Comparative Example 2 | Li-TFSI | none | - | 23.30±0.23 | 0.93±0.02 | 0.62±0.02 | 13.3±0.7 |
| Comparative Example 3 | methyltri-n-octylammonium TFSI | quatemary | - | 22.48±0.99 | 0.91±0.02 | 0.47±0.06 | 9.6±1.8 |
| Comparative Example 4 | 1-ethyl-3-methylimidazolium TFSI | imidazole | - | 24.32±0.63 | 0.86±0.03 | 0.54±0.10 | 11.3±2.5 |

Table 2

| | dopant material | degree of substitution of ammonium | synthesis method | reverse scan | | | |
|---|---|---|---|---|---|---|---|
| | | | | short circuit current density Jsc (mA/cm$^2$) | open circuit voltage Voc (V) | fill factor FF | conversion efficiency η (%) |
| Example 1 | n-octylammonium TFSI | primary | ion exchange | 23.46±0.55 | 1.05±0.01 | 0.75±0.01 | 18.48±0.64 |
| Example 2 | n-octylammonium TFSI | primary | neutralization | 26.44±0.15 | 1.15±0.01 | 0.80±0.01 | 24.25±0.34 |
| Example 3 | n-dodecylammonium TFSI | primary | neutralization | 25.99±0.41 | 1.07±0.02 | 0.72±0.02 | 19.12±1.31 |
| Example 4 | n-butylammonium TFSI | primary | neutralization | 26.26±0.44 | 1.07±0.03 | 0.76±0.01 | 21.61±0.65 |
| Example 5 | ethylammonium TFSI | primary | neutralization | 25.94±0.23 | 1.09±0.01 | 0.77±0.03 | 21.83±0.94 |
| Example 6 | 2-phenylethylammonium TFSI | primary | neutralization | 26.47±0.16 | 1.08±0.01 | 0.75±0.01 | 21.54±0.33 |
| Example 7 | N-methyl-n-octylammonium TFSI | secondary | neutralization | 25.24±0.27 | 1.04±0.01 | 0.68±0.02 | 19.46±0.67 |
| Example 8 | n-octylammonium FSI | primary | ion exchange | 25.43±0.16 | 1.01±0.01 | 0.76±0.02 | 17.87±0.75 |
| Comparative Example 1 | none | none | - | 15.79±1.92 | 1.04±0.01 | 0.68±0.02 | 2.89±0.97 |
| Comparative Example 2 | Li-TFSI | none | - | 23.21±0.20 | 0.97±0.01 | 0.73±0.01 | 16.54 ± 0.25 |
| Comparative Example 3 | methyltri-n-octylammonium TFSI | quatemary | - | 22.55±0.94 | 0.97±0.03 | 0.56±0.07 | 12.27±2.18 |
| Comparative Example 4 | 1-ethyl-3-methylimidazolium TFSI | imidazole | - | 24.59±0.18 | 0.95±0.02 | 0.69±0.07 | 16.18±1.76 |

It can be seen from the results shown in Table 1 and Table 2 that numerical values higher than those in Comparative Examples were obtained in the present Examples, in almost all of the short circuit current density, the open circuit voltage, the fill factor and the conversion efficiency, particularly in the open circuit voltage and the conversion efficiency, which are important performance factors of solar cells. In other words, improvements in the solar cell properties were observed in the present Examples. In particular, a marked effect was observed in the improvement of the open circuit voltage, which is thought to be due to the effect of a high reactivity of the organic ammonium cation included in the present ionic compound.

(Example 9)

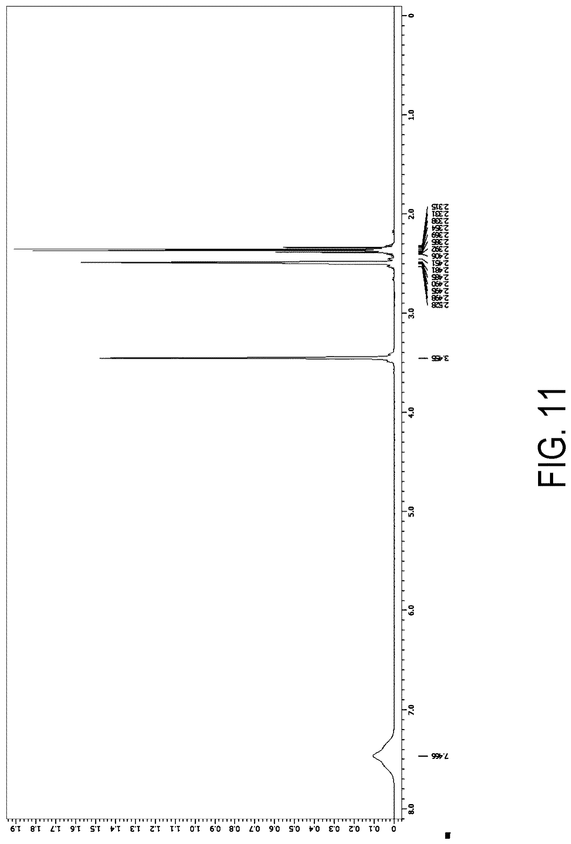

On the substrate on which the electron-transporting layer had been deposited in the same manner as in Example 1, a methylammonium lead iodide solution obtained by dissolving 1.50 mM of methylamine hydrogen iodide and 1.58 mM of lead iodide in a mixture ofN,N-dimethylformamide (manufactured by Sigma-Aldrich Co. LLC) and dimethyl sulfoxide (manufactured by Sigma-Aldrich Co. LLC) mixed at a volume ratio of 4:1 was spin-coated at 6,000 rpm for 10 seconds, and 0.2 mL of chlorobenzene (manufactured by Sigma-Aldrich Co. LLC) was dropped thereon 4 seconds after the start of the spin coating. After the spin coating described above, the sample was heated at 100°C for 10 minutes, and then heated at 110°C for 10 minutes, to form a thin perovskite layer. Thereafter, 4.8 mM of methylammonium TFSI synthesized by the following method, as a dopant, was added to a solution obtained by adding 14 mM of 4-tertbutylpyridine to a 10 mg/mL PTAA-toluene solution, and the resulting solution was deposited on the perovskite layer at 3,000 rpm. Thereafter, Au was deposited in the same manner as in Example 1, to produce a solar cell. The results are shown in Table 3-1 and Table 3-2.
The ionic compound as a dopant was prepared by the following method (acid-base neutralization method).
Ethanol (85 mL) was introduced into to a 300 mL four-neck flask, and the flask was immersed in an ice bath. To the flask, H-TFSI (12.0 g, 42.6 mmol, manufactured by Tokyo Chemical Industry Co., Ltd.) was added to prepare a homogeneous solution. Thereafter, methylamine (16.5 mL, 42.6 mmol) in ethanol (85mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at 40°C for 12 hours, and then at 60°C for two hours to obtain an ionic compound (methylammonium TFSI) in the form of a solid. The results of the $^1$H-NMR measurement of the resulting ionic compound are shown in FIG. 11. The melting point, as determined from the result of the measurement using the differential scanning calorimeter, was 43°C.

(Comparative Example 5)

A perovskite solar cell was produced in the same manner as in Example 9, except that Li-TFSI was used as the ionic compound as a dopant. The results are shown in Table 3-1 and Table 3-2.

[Table 3-1]

Table 3-1

| | dopant material | degree of substitution of ammonium | cation of perovskite material | forward scan | | | |
|---|---|---|---|---|---|---|---|
| | | | | short circuit current density Jsc (mA/cm$^2$) | open circuit voltage Voc (V) | fill factor FF | conversion efficiency $\eta$ (%) |
| Example 9 | methylammonium TFSI | primary | methylammonium | 21.72$\pm$1.59 | 1.06$\pm$0.02 | 0.43$\pm$0.07 | 10.06$\pm$2.12 |
| Comparative Example 5 | Li-TFSI | - | methylammonium | 22.23$\pm$1.35 | 1.03$\pm$0.01 | 0.30$\pm$0.06 | 6.99$\pm$1.52 |

[Table 3-2]

Table 3-2

| | dopant material | degree of substitution of ammonium | cation of perovskite material | reverse scan | | | |
|---|---|---|---|---|---|---|---|
| | | | | short circuit current density Jsc (mA/cm$^2$) | open circuit voltage Voc (V) | fill factor FF | conversion efficiency $\eta$ (%) |
| Example 9 | methylammonium TFSI | primary | methylammonium | 21.42$\pm$1.37 | 1.09$\pm$0.01 | 0.73$\pm$0.04 | 17.01$\pm$1.89 |
| Comparative Example 5 | Li-TFSI | - | methylammonium | 20.74$\pm$1.75 | 1.06$\pm$0.01 | 0.73$\pm$0.03 | 15.97$\pm$1.54 |

It can be seen from the results shown in Table 3-1 and Table 3-2 that numerical values higher than those in Comparative Examples were obtained in the present Examples, in almost all of the short circuit current density, the open circuit voltage, the fill factor and the conversion efficiency, particularly in the open circuit voltage and the conversion efficiency, which are important performance factors of solar cells. In other words, improvements in the solar cell properties were observed in the present Examples. In particular, a marked effect was observed in the improvement of the open circuit voltage, which is thought to be due to the effect of a high reactivity of the primary organic ammonium cation included in the present ionic compound. In Example 9, in particular, an improvement in performance by the methylammonium cation which is the same as the cation included in the perovskite material was observed. It can be seen that this leads to a less marked decrease in performance, even if the cation is diffused in the bulk of the perovskite layer when continuously used as a solar cell, making it advantageous also in an improvement in durability.

(Example 10)

A perovskite solar cell was produced in the same manner as in Example 1, except for the following. Specifically, the film formation of the perovskite layer performed in the Example 1 was carried out by spin coating a cesium formamidinium lead iodide solution obtained by dissolving 0.07 mM of cesium iodide, 1.0 mM of formamidine hydrogen iodide and 1.15 mM of lead iodide in a mixture of N,N-dimethylformamide (manufactured by Sigma-Aldrich Co. LLC) and dimethyl sulfoxide (manufactured by Sigma-Aldrich Co. LLC) mixed at a volume ratio of 4:1, at 6,000 rpm for 30 seconds, and by dropping 0.5 mL of diethyl ether (manufactured by FUJIFILM Wako Pure Chemical Corporation) thereon 10 seconds after the start of the spin coating. Further, in the film formation of the hole-transporting material, a solution obtained by adding a toluene solution containing 9.6 mM of the n-octylammonium TFSI synthesized in Example 2 and 7 mM of 4-tert-butylpyridine to 10 mg/mL of PTAA was used as the hole-transporting material solution, and the resulting solution was spin-coated on the perovskite layer at 3,000 rpm for 3 seconds to form a film. Except for the above, the perovskite solar cell was produced in the same manner as in Example 1. The results are shown in Table 4-1 and Table 4-2.

(Example 11)

A perovskite solar cell was produced in the same manner as in Example 10, except that a solution obtained by adding a toluene solution containing 4.8 mM of the phenylethylammonium synthesized in Example 6 and 7 mM of 4-tert-butylpyridine to 10 mg/mL of PTAA was used as the hole-transporting material solution, instead of the hole-transporting material solution prepared in Example 10. The results are shown in Table 4-1 and Table 4-2.

(Comparative Example 6)

A perovskite solar cell was produced in the same manner as in Example 10, except that a solution obtained by adding a toluene solution containing 4.8 mM of Li-TFSI and 7 mM of 4-tert-butylpyridine to 10 mg/mL of PTAA was used as the hole-transporting material solution, instead of the hole-transporting material solution prepared in Example 10. The results are shown in Table 4-1 and Table 4-2.

[Table 4-1]

Table 4-1

| | dopant material | degree of substitution of ammonium | forward scan | | | |
|---|---|---|---|---|---|---|
| | | | short circuit current density Jsc (mA/cm$^2$) | open circuit voltage Voc (V) | fill factor FF | conversion efficiency $\eta$ (%) |
| Example 10 | n-octylammonium TFSI | primary | 24.92 $\pm$ 0.21 | 0.97 $\pm$ 0.03 | 0.70 $\pm$ 0.03 | 17.05 $\pm$ 1.13 |
| Example 11 | phenylethylammonium TFSI | primary | 25.54 $\pm$ 0.15 | 0.97 $\pm$ 0.02 | 0.72 $\pm$ 0.02 | 17.96 $\pm$ 0.73 |

(continued)

| | dopant material | degree of substitution of ammonium | forward scan | | | |
|---|---|---|---|---|---|---|
| | | | short circuit current density Jsc (mA/cm$^2$) | open circuit voltage Voc (V) | fill factor FF | conversion efficiency η (%) |
| Comparative Example 6 | Li-TFSI | - | 25.37 ± 0.27 | 0.91 ± 0.03 | 0.63 ± 0.03 | 14.47 ± 1.04 |

[Table 4-2]

Table 4-2

| | dopant material | degree of substitution of ammonium | reverse scan | | | |
|---|---|---|---|---|---|---|
| | | | short circuit current density Jsc (mA/cm$^2$) | open circuit voltage Voc (V) | fill factor FF | conversion efficiency η (%) |
| Example 10 | n-octylammonium TFSI | primary | 24.92 ± 0.21 | 1.06 ± 0.01 | 0.78 ± 0.03 | 20.67 ± 0.83 |
| Example 11 | phenylethylammonium TFSI | primary | 25.55 ± 0.16 | 1.04 ± 0.01 | 0.80 ± 0.01 | 21.15 ± 0.51 |
| Comparative Example 6 | Li-TFSI | - | 25.37 ± 0.28 | 1.00 ± 0.02 | 0.74 ± 0.02 | 18.83 ± 0.88 |

It can be seen from the results shown in Table 4-1 and Table 4-2 that numerical values higher than those in Comparative Examples were obtained in the present Examples, in almost all of the short circuit current density, the open circuit voltage, the fill factor and the conversion efficiency, particularly in the open circuit voltage and the conversion efficiency, which are important performance factors of solar cells. In other words, improvements in the solar cell properties were observed in the present Examples. In particular, a marked effect was observed in the improvement of the open circuit voltage, which is thought to be due to the effect of a high reactivity of the organic ammonium cation included in the present ionic compound. In Example 11, in particular, it has been found out that properties better than those in Example 10 are shown, which is thought to be due to an improvement in the adhesion between the hole-transporting material and the perovskite layer, as a result of the fact that the organic moiety of the organic ammonium contains an aromatic moiety.

(Examples 12 to 21)

(Water Contact Angle)

In Example 12, the same perovskite layer as that in Example 1 and the same hole-transporting layer as that in Example 1 were deposited on a non-alkali glass substrate, in the same manner as the production of the perovskite solar cell described above. Thereafter, the operation of washing the surface of the thus prepared sample with 1 mL of chlorobenzene was repeated 40 times, followed by drying with dry air, to obtain a perovskite layer sample from which the hole-transporting layer had been removed. The water contact angle of the resulting sample was measured. In Example 13, a sample was prepared and the water contact angle of the perovskite layer was measured in the same manner as in Example 12, except that the same hole-transporting layer as that in Example 3 was formed. In Example 14, a sample was prepared and the water contact angle of the perovskite layer was measured in the same manner as in Example 12, except that the same hole-transporting layer as that in Example 4 was formed. In Example 15, a sample was prepared and the water contact angle of the perovskite layer was measured in the same manner as in Example 12, except that the same hole-transporting layer as that in Example 5 was formed. In Example 16, a sample was prepared and the water contact angle of the perovskite layer was measured in the same manner as in Example 12, except that the same hole-transporting layer as that in Example 7 was formed. In Example 17, a sample was prepared and the water contact angle of the perovskite layer was measured in the same manner as in Example 12, except that the same hole-transporting layer as that in Example 8 was formed. The results are shown in Table 5.

In Example 18, a sample was prepared and the water contact angle of the perovskite layer was measured in the same manner as in Example 12, except that the ionic compound was prepared by the following method, and that a solution obtained by dissolving 70 mM of Spiro-OMeTAD (manufactured by Nippon Fine Chemical Co., Ltd.), 24 mM of n-octylammonium PFSI prepared by the following method and 204 mM of the t-butylpyridine in chlorobenzene, was used as the hole-transporting layer precursor solution. The result is shown in Table 5.

The ionic compound as a dopant was prepared by the following method (ion exchange method).

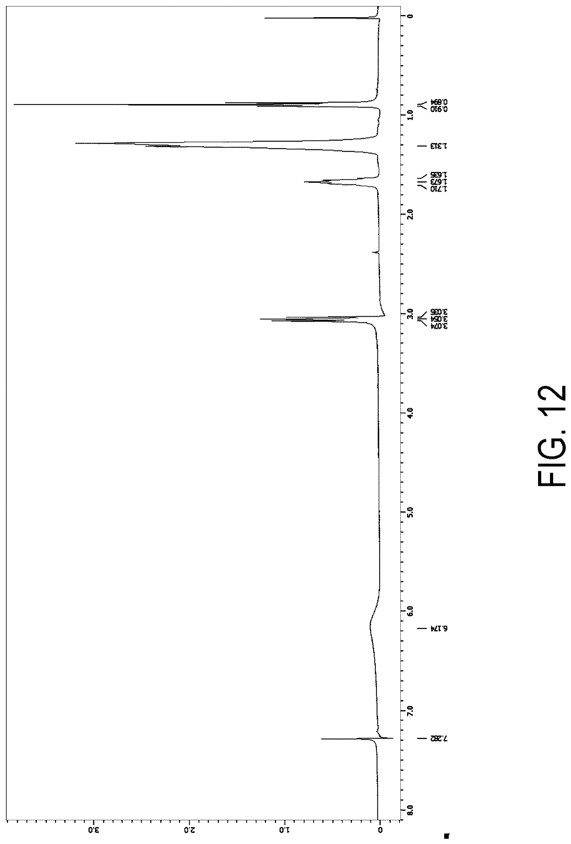

Under a nitrogen gas stream, 3.31 g (20 mmol) of octylamine hydrochloride (manufactured by Tokyo Chemical Industry Co., Ltd.) was weighed into a 200 mL two-necked flask, and dissolved in 20 mL of pure water. To the resulting aqueous solution, 20 mL of an aqueous solution of 7.74 g (20 mmol) of lithium bis(pentafluoroethanesulfonyl)imide (manufactured by Tokyo Chemical Industry Co., Ltd.) was added, followed by stirring at room temperature for two hours. Two hours later, a chloroform solution (manufactured by Kishida Chemical Co., Ltd.) was added for liquid separation, and the chloroform layer was washed twice with ultrapure water. Thereafter, chloroform was removed from the chloroform solution at 40°C using an evaporator, followed by vacuum drying at room temperature, to obtain an ionic liquid of n-octylammonium PFSI. The results of the $^1$H-NMR measurement of the resulting ionic compound are shown in FIG. 12. The melting point, as determined from the result of the measurement using the differential scanning calorimeter, was 7°C. Further, the lithium content was less than 0.2 ppm by mass.

In Example 19, a sample was prepared and the water contact angle of the perovskite layer was measured in the same manner as in Example 12, except that the ionic compound was prepared by the following method, and that a solution obtained by dissolving 70 mM of Spiro-OMeTAD (manufactured by Nippon Fine Chemical Co., Ltd.), 24 mM of n-octylammonium NFSI prepared by the following method and 204 mM of the t-butylpyridine in chlorobenzene, was used as the hole-transporting layer precursor solution. The result is shown in Table 5.

The ionic compound as a dopant was prepared by the following method (ion exchange method).

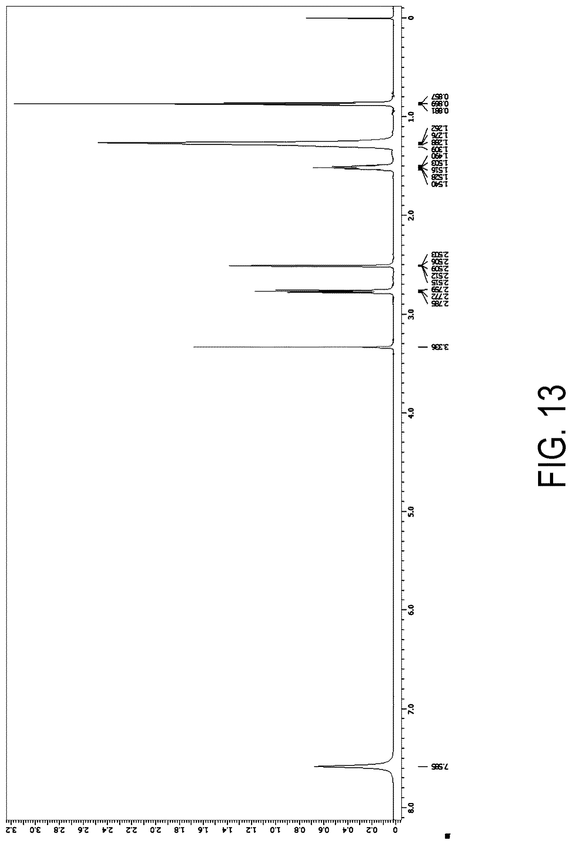

Under a nitrogen gas stream, 3.31 g (20 mmol) of octylamine hydrochloride (manufactured by Tokyo Chemical Industry Co., Ltd.) was weighed into a 200 mL two-necked flask, and dissolved in 20 mL of pure water. To the resulting aqueous solution, 20 mL of an aqueous solution of 11.74 g (20 mmol) of lithium bis(nonafluorobutanesulfonyl)imide (manufactured by Tokyo Chemical Industry Co., Ltd.) was added, followed by stirring at room temperature for two hours. Two hours later, a chloroform solution (manufactured by Kishida Chemical Co., Ltd.) was added for liquid separation, and the chloroform layer was washed twice with ultrapure water. Thereafter, chloroform was removed from the chloroform solution at 40°C using an evaporator, followed by vacuum drying at room temperature, to obtain an ionic liquid of n-octylammonium NFSI. The results of the $^1$H-NMR measurement of the resulting ionic compound are shown in FIG. 13. The melting point, as determined from the result of the measurement using the differential scanning calorimeter, was 141°C. Further, the lithium content was 0.79 ppm by mass.

In Example 20, a sample was prepared and the water contact angle of the perovskite layer was measured in the same manner as in Example 12, except that the ionic compound was prepared by the following method, and that a solution obtained by dissolving 70 mM of Spiro-OMeTAD (manufactured by Nippon Fine Chemical Co., Ltd.), 24 mM of n,n-dimethyl-n-octylammonium TFSI prepared by the following method and 204 mM of the t-butylpyridine in chlorobenzene, was used as the hole-transporting layer precursor solution. The result is shown in Table 5.

The ionic compound as a dopant was prepared by the following method (acid-base neutralization method).

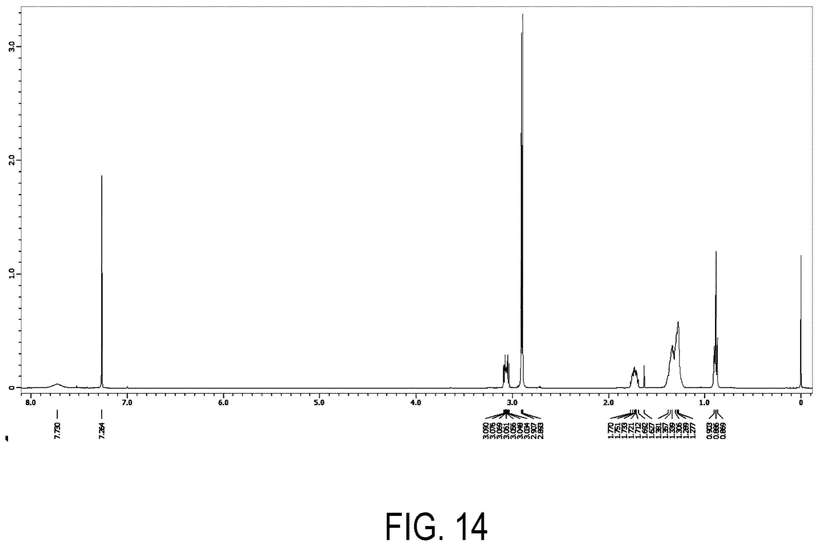

Ethanol (83 mL) was introduced into a 500 mL four-neck flask, and the flask was immersed in an ice bath. To the flask, H-TFSI (14.30 g, 50.9 mmol, manufactured by Tokyo Chemical Industry Co., Ltd.) was added to prepare a homogeneous solution. Thereafter, n,n-dimethyl-n-octylamine (8.00 g, 50.9 mmol) in ethanol (100 mL) was added dropwise using a funnel. After the completion of the dropwise addition of the entire amount thereof over 30 minutes, the interior of the funnel was washed with a small amount of ethanol. Then the flask was stirred for two hours while keeping the flask immersed in the ice bath. Thereafter, the resulting reaction liquid was transferred into an eggplant flask, and concentrated under reduced pressure at 60°C for 12 hours, to obtain an ionic liquid of n,n-dimethyl-n-octylammonium TFSI. The results of the $^1$H-NMR measurement of the resulting ionic compound are shown in FIG. 14. The melting point, as determined from the result of the measurement using the differential scanning calorimeter, was -35°C.

In Example 21, the same perovskite layer as that in Example 11 and the same hole-transporting layer as that in Example 11 were deposited on a non-alkali glass substrate, in the same manner as the production of the perovskite solar cell described above. Thereafter, the hole-transporting layer was removed by peeling off the hole-transporting layer using a tape, and the water contact angle of perovskite layer was evaluated in the same manner as in Example 12. The result is shown in Table 5.

(Comparative Examples 7 to 9)

In Comparative Example 7, a sample was prepared, and the water contact angle of the perovskite layer was measured in the same manner as in Example 12, except that the same hole-transporting layer as that in Comparative Example 2 was formed. In Comparative Example 8, a sample was prepared, and the water contact angle of the perovskite layer was measured in the same manner as in Example 12, except that the same hole-transporting layer as that in Comparative Example 4 was formed. In Comparative Example 9, a sample was prepared, and the water contact angle of the perovskite layer was measured in the same manner as in Example 12, except that the same hole-transporting material as that in Comparative Example 6 was used. The results are shown in Table 5.

[Table 5]

Table 5

| | | dopant material | degree of substitution of ammonium | water contact angle (degree) |
|---|---|---|---|---|
| | Example 12 | n-octylammonium TFSI | primary | 80.2 ± 2.0 |
| | Example 13 | n-dodecylammonium TFSI | primary | 93.9 ± 2.7 |
| | Example 14 | n-butylammonium TFSI | primary | 68.9 ± 1.7 |
| | Example 15 | ethylammonium TFSI | primary | 57.0 ± 1.7 |
| | Example 16 | N-methyl-n-octylammonium TFSI | secondary | 78.6 ± 3.3 |
| | Example 17 | n-octylammonium FSI | primary | 77.5 ± 0.5 |
| | Example 18 | n-octylammonium PFSI | primary | 69.7 ± 1.4 |
| | Example 19 | n-octylammonium NFSI | primary | 78.4 ± 9.6 |
| | Example 20 | N,N-dimethyl-n-octylammonium TFSI | tertiary | 64.0 ± 5.6 |
| | Example 21 | 2-phenylethylammonium TFSI | primary | 60.3 = 1.7 |
| | Comparative Example 7 | Li-TFSI | none | 45.6 ± 2.0 |
| | Comparative Example 8 | 1-ethyl-3-methylimidazolium TFSI | imidazole | 50.4 ± 1.4 |
| | Comparative Example 9 | Li-TFSI | none | 50.1 ± 0.9 |

A higher value of the water contact angle indicates a higher hydrophobicity, and thus, a higher water resistance. As can be seen from the results in Table 5, the dopants of the present Examples showed a higher water resistance as compared to those of Comparative Examples. This has shown that incorporating the ionic compound of the present Example into a hole-transporting material precursor solution makes it possible to suitably hydrophobize the surface of the perovskite layer, namely, and to impart water resistance to the surface. Therefore, the ionic compounds of the present Examples improve the weather resistance, the moisture resistance and the like, and thus can be used in a wide range of applications, such as solar cells and photosensors.

(Examples 22 to 25)

In Example 22, the perovskite solar cell produced in Example 1 was subjected to a durability test under the conditions of a temperature of 30°C and a humidity of 50% for 500 hours, to examine the remaining solar cell performance after the test, and the result thereof is shown. The remaining solar cell performance was determined by (solar energy conversion efficiency of solar cell after the test)/(solar energy conversion efficiency of solar cell before the test). In Example 23, a solar cell was produced in the same manner as in Example 3, and the sample evaluation after the durability test was carried out in the same manner as in Example 22. In Example 24, a solar cell was produced in the same manner as in Example 1 except that the same hole-transporting material solution as that in Example 18 was used, and the sample evaluation after the durability test was carried out in the same manner as in Example 22. In Example 25, a solar cell was produced in the same manner as in Example 1 except that the same hole-transporting material solution as that in Example 20 was used, and the sample evaluation after the durability test was carried out in the same manner as in Example 22. The evaluation results are shown in Table 6.

(Comparative Example 10)

The perovskite solar cell produced in Comparative Example 2 was subjected to the same test as that in Example 22. The result thereof is shown in Table 6.

[Table 6]

Table 6

| | dopant material | number of carbon atom | degree of substitution of ammonium | anion | relative performance after 500 h compared to the initial performance (%) |
|---|---|---|---|---|---|
| Example 22 | n-octylammonium TFSI | 8 | primary | TFSI | 91 |
| Example 23 | n-dodecylammonium TFSI | 12 | primary | TFSI | 101 |
| Example 24 | n-octylammonium PFSI | 8 | primary | PFSI | 105 |
| Example 25 | N,N-dimethyl-n-octylammonium TFSI | 8 | tertiary | TFSI | 95 |
| Comparative Example 10 | Li-TFSI | 0 | none | TFSI | 78 |

(Examples 26 to 28)

In Example 26, the perovskite solar cell produced in Example 1 was subjected to a durability test under the conditions of a temperature of 30°C and a humidity of 50% for 1,000 hours, to examine the remaining solar cell performance after the test. The remaining solar cell performance was calculated in the same manner as in Example 22. In Example 27, a solar cell was produced in the same manner as in Example 3, and the sample evaluation after the durability test was carried out in the same manner as in Example 26. In Example 28, a solar cell was produced in the same manner as in Example 11, and the sample evaluation after the durability test was carried out in the same manner as in Example 26. The evaluation results are shown in Table 7.

(Comparative Examples 11 and 12)

In Comparative Example 11, the perovskite solar cell produced in Comparative Example 2 was subjected to the same test as that in Example 26. In Comparative Example 12, the perovskite solar cell produced in Comparative Example 6 was subjected to the same test as that in Example 26. The results thereof are shown in Table 7.

[Table 7]

Table 7

| | dopant material | number of carbon atom in organic moiety | degree of substitution of ammonium | relative performance after 1000 h compared to the initial performance |
|---|---|---|---|---|
| Example 26 | n-octylammonium TFSI | 8 | primary | 85% |

(continued)

| | dopant material | number of carbon atom in organic moiety | degree of substitution of ammonium | relative performance after 1000 h compared to the initial performance |
|---|---|---|---|---|
| Example 27 | n-dodecylammonium TFSI | 12 | primary | 98% |
| Example 28 | phenylethylammonium TFSI | 8 | primary | 94% |
| Comparative Example 11 | Li-TFSI | 0 | none | 67% |
| Comparative Example 12 | Li-TFSI | 0 | none | 44% |

As can be seen from the results in Table 6 and Table 7, the solar cells in which the dopants of the present Examples had been used showed a higher remaining solar cell performance as compared to those in which the dopants of Comparative Examples had been used. Further, solar cells in which n-dodecylammonium having 12 carbon atoms had been used showed an even higher remaining solar cell performance as compared to those in which n-octylammonium having 8 carbon atoms had been used. The comparison between Example 22 and Example 24 in Table 6 has shown that increasing the hydrophobicity of the hole-transporting material by using the PFSI anion having a larger number of carbon atoms and fluorine atoms than those in the TFSI anion, is more effective in improving the durability as a solar cell. Further, the comparison between Example 22 and Example 25 in Table 6 has shown that increasing the degree of substitution of the organic ammonium from that in a primary organic ammonium, and/or increasing the number of carbon atoms in the organic moiety of the organic ammonium, is effective in improving the durability. In addition to the above, the comparison between Example 28 and Comparative Example 12 in Table 7 has shown that one containing a phenylethyl group having an aromatic moiety is also effective in improving the durability of the resulting perovskite solar cell. In other words, it can be said that such arrangements allow for imparting solar cell properties with improved weather resistance and moisture resistance and the like, as well as a long life. This is thought to be due to the surface of the perovskite which is hydrophilic being hydrophobized by the ionic compounds of the present Examples, thereby imparting the water resistance. Therefore, the ionic compounds of the present Examples improve the weather resistance, the moisture resistance and the like, and thus can be used in a wide range of applications, such as solar cells and photosensors.

(Examples 29 to 32)

In Example 29, the viscosity of the n-octylammonium TFSI synthesized by the ion exchange method in Example 1 was measured using a Type E viscometer (TV-25, manufactured by Toki Sangyo Co., Ltd.) under the temperature condition of 25°C. In Example 30, the viscosity of the n-octylammonium TFSI synthesized by the neutralization method in Example 2 was evaluated in the same manner as in Example 29. In Example 31, the viscosity of the n-methyl-n-octylammonium TFSI synthesized in Example 7 was evaluated in the same manner as in Example 29. In Example 32, the viscosity of the n-octylammonium FSI synthesized in Example 8 was evaluated in the same manner as in Example 29. In Example 33, the viscosity of the n,n-dimethyl-n-octylammonium TFSI synthesized in Example 20 was evaluated in the same manner as in Example 29. The results thereof are shown in Table 8.

[Table 8]

Table 8

| | material | synthesis method | degree of substitution of ammonium | anion | viscosity (mPa s) |
|---|---|---|---|---|---|
| Example 29 | n-octylammonium TFSI | ion exchange | primary | TFSI | 260 |
| Example 30 | n-octylammonium TFSI | neutralization | primary | TFSI | 572 |
| Example 31 | N-methyl-n-octylammonium TFSI | neutralization | secondary | TFSI | 128 |
| Example 32 | n-octylammonium FSI | ion exchange | primary | FSI | 25 |

(continued)

| | material | synthesis method | degree of substitution of ammonium | anion | viscosity (mPa s) |
|---|---|---|---|---|---|
| Example 33 | N,N-dimethyl-n-octylammonium TFSI | neutralization | tertiary | TFSI | 94 |

It can be seen from the comparison between the results of Example 29 and Example 30 in Table 8 that the ionic compound synthesized by the ion echange method showed a viscosity lower than that synthesized by the neutralization method. The above results have shown that the ion exchange method allows for faster mixing in the preparation step of the raw material solution, and that it is advantageous when mixed with and diffused into another material(s). This is thought to be due to the presence of the lithium cation in the ionic compound synthesized by the ion exchange method. It has been shown that the incorporation of an alkali metal cation into an ionic compound makes it more advantageous in the production process.

Further, the comparison between Examples 30, 31 and 33 has shown that increasing the degree of substitution of the organic ammonium in the cation of the ionic liquid decreases the viscosity, and allows for faster mixing in the preparation step of the raw material solution, and that it is more advantageous when mixed with and diffused into another material(s). In addition, the comparison between Example 29 and Example 32 has shown that using the FSI anion instead of the TFSI anion decreases the viscosity, and allows for faster mixing in the preparation step of the raw material solution, and that it is more advantageous when mixed with and diffused into another material(s).

(Examples 34 to 36)

In Example 34, a perovskite solar cell was produced in the same manner as in Example 2, except that the amount of dopant was increased to twice the amount of that in Example 2, which was 48 mM. Thereafter, the performance of the resulting solar cell was compared with that produced in Example 2 taken as the standard, and the relative performance when doped with twice the amount of dopant was calculated as follows.

(Relative performance when doped with twice the amount) = (solar energy conversion efficiency when doped with twice the amount)/(solar energy conversion efficiency in the Example taken as the standard)

In Example 35, a solar cell was produced in the same manner as in Example 4, except that the amount of dopant was changed to 36 mM. Thereafter, the relative performance was evaluated in the same manner as in Example 34, except that the performance of the solar cell produced in Example 4 was taken as the standard. In Example 36, a solar cell was produced in the same manner as in Example 5, except that the amount of dopant was changed to 36 mM. Thereafter, the relative performance was evaluated in the same manner as in Example 34, except that the performance of the solar cell produced in Example 5 was taken as the standard. The results are shown in Table 9.

[Table 9]

Table 9

| | dopant material | number of carbon atom in organic moiety | relative performance when doped with twice the optimal amount |
|---|---|---|---|
| Example 34 | n-octylammonium TFSI | 8 | 90 |
| Example 35 | n-butylammonium TFSI | 4 | 93 |
| Example 36 | ethylammonium TFSI | 2 | 100 |

The fact that the dopant concentration dependence of the solar cell performance is low leads to an excellent uniformity in the cell production, and is industrially advantageous, for example, in enlargement of the cell area, and the like. The results in Table 6 have shown that the dopant concentration dependence of the solar cell performance is further decreased, by decreasing the number of carbon atoms in the organic moiety from 8 to 4, and further to 2.

(Examples 37 and 38)

In Example 37, the adhesion force of the hole-transporting layer to the perovskite layer was evaluated for a sample in which a perovskite layer and a hole-transporting layer were deposited in the same manner as in Example 10, by a 180-degree tape peeling test of the hole-transporting layer. The measurement was carried out using TENSILON UCT-Type 5T (manufactured by Orientec Co., Ltd.) as the apparatus, and a Kapton tape, at a crosshead speed of 100 mm/min. The average adhesion force was defined as the arithmetic mean value of the measured values of the adhesion force within the range of crosshead length from 10 to 50 mm, per tape width. In Example 38, the adhesion force was evaluated in the same manner as in Example 37, for a sample in which a perovskite layer and a hole-transporting layer were deposited in the same manner as in Example 11. The results are shown in Table 10.

[Table 10]

Table 10

|  | dopant material | average adhesion force (N cm$^{-1}$) |
|---|---|---|
| Example 37 | n-octylammonium TFSI | 0.03 ± 0.01 |
| Example 38 | phenylethylammonium TFSI | 1.8 ± 0.1 |

The results in Table 10 have shown that it is more advantageous in improving the adhesion between the hole-transporting material and the perovskite layer when the organic moiety contains an aromatic moiety.

(Example 39)

A perovskite solar cell was produced in the same manner as in Example 10, except that 4-tert-butylpyridine was not added to the hole-transporting material solution. The solar cell performance of the thus produced solar cell after being subjected to a heat resistance test at 85°C for 68 hours in nitrogen was evaluated in the same manner as the initial performance, and the relative performance compared to the initial performance was calculated. The result is shown in Table 11.

(Example 40)

A perovskite solar cell was produced in the same manner as in Example 10, except that 4 mM of 4-tert-butylpyridine was added to the hole-transporting material solution, and the relative performance was evaluated in the same manner as in Example 39. The result is shown in Table 11.

(Example 41)

A perovskite solar cell was produced in the same manner as in Example 10, and the relative performance was evaluated in the same manner as in Example 39. The result is shown in Table 11.

(Example 42)

A perovskite solar cell was produced in the same manner as in Example 10, except that 14 mM of 4-tert-butylpyridine was added to the hole-transporting material solution, and the relative performance was evaluated in the same manner as in Example 39. The result is shown in Table 11.

(Example 43)

A perovskite solar cell was produced in the same manner as in Example 10, except that 28 mM of 4-tert-butylpyridine was added to the hole-transporting material solution, and the relative performance was evaluated in the same manner as in Example 39. The result is shown in Table 11.

[Table 11]

**Table 11**

|  | addition amount of 4-tert-butylpyridine (mM) | relative performance after heat resistance test compared to the initial performance |
|---|---|---|
| Example 39 | 0 | 74 |
| Example 40 | 4 | 83 |
| Example 41 | 7 | 94 |
| Example 42 | 14 | 94 |
| Example 43 | 28 | 97 |

The results in Table 11 have shown that the heat resistance of the resulting solar cell is improved by adding 4-tert-butylpyridine to the hole-transporting material, along with the ionic compound according to the present embodiment. This is a phenomenon contrary to the findings so far, and is a surprising result.

These Examples have shown that the ionic compound according to the present embodiment is capable of improving the solar cell performance and/or the water resistance, because the organic ammonium cation included therein has a high reactivity. Therefore, it can be said that the ionic compounds of the present Examples can be used in a wide range of applications that utilize reactivity, such as electric materials, reagents, solvents for organic synthesis, solvents for separation and extraction, gas absorbents and catalysts.

As described above, the ionic compound according to one embodiment, the hole-transporting material according to one embodiment, and the perovskite solar cell according to one embodiment, are as follows.

(1) An ionic compound including a molecular cation and a molecular anion,

wherein the molecular cation includes at least one organic ammonium selected from the group consisting of a primary organic ammonium, a secondary organic ammonium, and a tertiary organic ammonium,
wherein the organic ammonium has an organic moiety having 1 to 20 carbon atoms, and
wherein the molecular anion includes a fluorine-containing bis(sulfonyl)imide.

(2) The ionic compound according to (1), wherein the molecular cation includes the primary organic ammonium.
(3) The ionic compound according to (1) or (2), wherein the fluorine-containing bis(sulfonyl)imide is bis(trifluoromethanesulfonyl)imide.
(4) The ionic compound according to any one of (1) to (3), wherein the organic moiety of the organic ammonium has 2 to 12 carbon atoms.
(5) The ionic compound according to any one of (1) to (4), wherein the organic moiety of the organic ammonium includes a saturated hydrocarbon.
(6) The ionic compound according to (5), wherein the saturated hydrocarbon is a linear saturated hydrocarbon.
(7) The ionic compound according to any one of (1) to (5), wherein the organic moiety of the organic ammonium includes a cyclic aliphatic hydrocarbon.
(8) The ionic compound according to any one of (1) to (4), wherein the organic moiety of the organic ammonium includes an aromatic hydrocarbon.
(9) The ionic compound according to any one of (1) to (4) and (8), wherein the organic ammonium is 2-phenylethylammonium.
(10) The ionic compound according to any one of (1) to (8), wherein the organic moiety of the organic ammonium includes at least one functional group selected from the group consisting of a secondary ammonium, a tertiary ammonium, a quaternary ammonium, a thiol group, a carboxy group, and a hydroxy group.
(11) The ionic compound according to any one of (1) to (8) and (10), wherein the organic moiety of the organic ammonium includes a fluorocarbon.
(12) The ionic compound according to (11), wherein a carbon atom bonded to a nitrogen atom of the organic ammonium has a $CH_2$ structure.
(13) The ionic compound according to (11), wherein the organic moiety of the organic ammonium includes a perfluoroalkyl group.
(14) The ionic compound according to any one of (1) to (13), wherein the ionic compound is an ionic liquid having a melting point of 100°C or lower.

(15) A hole-transporting material containing the ionic compound according to any one of (1) to (14).

(16) The hole-transporting material according to (15), wherein the hole-transporting material contains a pyridine compound.

(17) The hole-transporting material according to (16), wherein the pyridine compound is 4-tert-butylpyridine.

(18) The hole-transporting material according to any one of (15) to (17), wherein the hole-transporting material contains Spiro-OMeTAD.

(19) A perovskite solar cell including:

a perovskite layer; and
a hole-transporting layer formed on the perovskite layer,

wherein the hole-transporting layer contains the hole-transporting material according to any one of (15) to (19).

(20) The perovskite solar cell according to (19),
wherein, when the hole-transporting layer is removed and the water contact angle of the exposed perovskite layer is measured, the water contact angle is 55.0° or more.

(21) The perovskite solar cell according to (19) or (20),
wherein the hole-transporting material contains poly[bis(4-phenyl)(2,4,6-trimethylphenyl)amine], and the average adhesion force of the hole-transporting layer to the perovskite layer is 1.0 N·cm$^{-1}$ or more.

(22) A method of producing the ionic compound according to any one of (1) to (14), the method including steps of:

ion-exchanging a halide of an organic ammonium having an organic moiety having 1 to 20 carbon atoms, with an alkali metal salt of a fluorine-containing bis(sulfonyl)imide; and
extracting the ionic compound obtained by the ion exchange.

(23) The method of producing the ionic compound, according to (22), wherein the extraction is performed using chloroform, in the step of extracting the ionic compound.

(24) The method of producing the ionic compound, according to (22) or (23), wherein a halogen ion contained in the halide of the organic ammonium is a chlorine ion.

(25) The method of producing the ionic compound, according to any one of (22) to (24), wherein an alkali metal contained in the alkali metal salt of the fluorine-containing bis(sulfonyl)imide is lithium.

(26) A method of producing the ionic compound according to any one of (1) to (14), the method including a step of neutralizing an organic amine having an organic moiety having 1 to 20 carbon atoms, with an acid of a fluorine-containing bis(sulfonyl)imide.

(27) The method of producing the ionic compound, according to any one of (22) to (26), wherein the organic moiety of the organic amine has 2 to 12 carbon atoms.

(28) A method of producing a hole-transporting material, the method including a step of mixing the ionic compound according to any one of (1) to (14), and a raw material compound of the hole-transporting material.

(29) The method of producing the hole-transporting material according to (28), wherein at least Spiro-OMeTAD is used as the raw material compound of the hole-transporting material.

(30) A method of producing a perovskite solar cell by layering an electron-transporting layer, a perovskite layer and a hole-transporting layer between an electrically conductive substrate and an electrode,

wherein the method of producing the perovskite solar cell includes a step of forming the hole-transporting layer, and
wherein the step of forming the hole-transporting layer includes a step of forming a film on the perovskite layer, using the hole-transporting material according to any one of (15) to (18).

INDUSTRIAL APPLICABILITY

The present disclosure can be used in various fields, such as the fields of reagents, solvents for organic synthesis, solvents for separation and extraction, gas absorbents, catalysts, and the like, in addition to electric materials. In the field of electric materials, the present disclosure can be used in various applications, such as solar cell materials, photosensors, luminescent materials, lithium-ion secondary batteries, and the like.

DESCRIPTION OF SYMBOLS

1      perovskite solar cell
3      electrically conductive substrate
5      electron-transporting layer
7      perovskite layer
9      hole-transporting layer
11     electrode

**Claims**

1. An ionic compound comprising a molecular cation and a molecular anion,

   wherein the molecular cation comprises at least one organic ammonium selected from the group consisting of a primary organic ammonium, a secondary organic ammonium, and a tertiary organic ammonium,
   wherein the organic ammonium comprises an organic moiety having 1 to 20 carbon atoms, and
   wherein the molecular anion comprises a fluorine-containing bis(sulfonyl)imide.

2. The ionic compound according to claim 1, wherein the molecular cation comprises the primary organic ammonium.

3. The ionic compound according to claim 1 or 2, wherein the fluorine-containing bis(sulfonyl)imide is bis(trifluoromethanesulfonyl)imide.

4. The ionic compound according to any one of claims 1 to 3, wherein the organic moiety of the organic ammonium has 2 to 12 carbon atoms.

5. The ionic compound according to any one of claims 1 to 4, wherein the organic moiety of the organic ammonium comprises a saturated hydrocarbon.

6. The ionic compound according to claim 5, wherein the saturated hydrocarbon is a linear saturated hydrocarbon.

7. The ionic compound according to any one of claims 1 to 5, wherein the organic moiety of the organic ammonium comprises a cyclic aliphatic hydrocarbon.

8. The ionic compound according to any one of claims 1 to 4, wherein the organic moiety of the organic ammonium comprises an aromatic hydrocarbon.

9. The ionic compound according to any one of claims 1 to 4 and 8, wherein the organic ammonium is 2-phenylethylammonium.

10. The ionic compound according to any one of claims 1 to 8, wherein the organic moiety of the organic ammonium comprises at least one functional group selected from the group consisting of a secondary ammonium, a tertiary ammonium, a thiol group, a carboxy group, and a hydroxy group.

11. The ionic compound according to any one of claims 1 to 8 and 10, wherein the organic moiety of the organic ammonium comprises a fluorocarbon.

12. The ionic compound according to claim 11, wherein a carbon atom bonded to a nitrogen atom of the organic ammonium has a $CH_2$ structure.

13. The ionic compound according to claim 11, wherein the organic moiety of the organic ammonium comprises a perfluoroalkyl group.

14. The ionic compound according to any one of claims 1 to 13, wherein the ionic compound is an ionic liquid having a melting point of 100°C or lower.

15. A hole-transporting material comprising the ionic compound according to any one of claims 1 to 14.

16. The hole-transporting material according to claim 15, wherein the hole-transporting material comprises a pyridine compound.

17. The hole-transporting material according to claim 16, wherein the pyridine compound is 4-tert-butylpyridine.

18. The hole-transporting material according to any one of claims 15 to 17, wherein the hole-transporting material comprises Spiro-OMeTAD.

19. A perovskite solar cell comprising:

   a perovskite layer; and
   a hole-transporting layer formed on the perovskite layer,

   wherein the hole-transporting layer comprises the hole-transporting material according to any one of claims 15 to 18.

20. The perovskite solar cell according to claim 19,
   wherein, when the hole-transporting layer is removed and the water contact angle of the exposed perovskite layer is measured, the water contact angle is 55.0° or more.

21. The perovskite solar cell according to claim 19 or 20,
   wherein the hole-transporting material comprises poly[bis(4-phenyl)(2,4,6-trimethylphenyl)amine], and the average adhesion force of the hole-transporting layer to the perovskite layer is 1.0 N·cm$^{-1}$ or more.

22. A method of producing the ionic compound according to any one of claims 1 to 14, the method comprising steps of:

   ion-exchanging a halide of an organic ammonium comprising an organic moiety having 1 to 20 carbon atoms, with an alkali metal salt of a fluorine-containing bis(sulfonyl)imide; and
   extracting the ionic compound obtained by the ion exchange.

23. The method of producing the ionic compound, according to claim 22, wherein the extraction is performed using chloroform, in the step of extracting the ionic compound.

24. The method of producing the ionic compound, according to claim 22 or 23, wherein a halogen ion contained in the halide of the organic ammonium is a chlorine ion.

25. The method of producing the ionic compound, according to any one of claims 22 to 24, wherein an alkali metal contained in the alkali metal salt of the fluorine-containing bis(sulfonyl)imide is lithium.

26. A method of producing the ionic compound according to any one of claims 1 to 14, the method comprising a step of neutralizing an organic amine comprising an organic moiety having 1 to 20 carbon atoms, with an acid of a fluorine-containing bis(sulfonyl)imide.

27. The method of producing the ionic compound, according to any one of claims 22 to 26, wherein the organic moiety of the organic amine has 2 to 12 carbon atoms.

28. A method of producing a hole-transporting material, the method comprising a step of mixing the ionic compound according to any one of claims 1 to 14, and a raw material compound of the hole-transporting material.

29. The method of producing the hole-transporting material according to claim 28, wherein at least Spiro-OMeTAD is used as the raw material compound of the hole-transporting material.

30. A method of producing a perovskite solar cell by layering an electron-transporting layer, a perovskite layer and a hole-transporting layer between an electrically conductive substrate and an electrode,

   wherein the method of producing the perovskite solar cell comprises a step of forming the hole-transporting layer, and
   wherein the step of forming the hole-transporting layer comprises a step of forming a film on the perovskite layer, using the hole-transporting material according to any one of claims 15 to 18.

FIG. 1

FIG. 2

EP 4 660 180 A1

FIG. 3

-78.57

| -200.0 | -190.0 | -180.0 | -170.0 | -160.0 | -150.0 | -140.0 | -130.0 | -120.0 | -110.0 | -100.0 | -90.0 | -80.0 | -70.0 | -60.0 | -50.0 | -40.0 | -30.0 | -20.0 | -10.0 | 0 |

0    10.0    20.0    30.0    40.0    50.0

EP 4 660 180 A1

40

FIG. 4

FIG. 5

FIG. 6

FIG. 7

FIG. 8

FIG. 9

FIG. 10

FIG. 11

FIG. 12

FIG. 13

FIG. 14

## INTERNATIONAL SEARCH REPORT

International application No.

**PCT/JP2024/002706**

### A. CLASSIFICATION OF SUBJECT MATTER

*C07C 211/63*(2006.01)i; *C07C 303/40*(2006.01)i; *C07C 311/48*(2006.01)i; *H10K 30/40*(2023.01)i; *H10K 30/50*(2023.01)i; *H10K 30/86*(2023.01)i; *H10K 50/135*(2023.01)i; *H10K 85/50*(2023.01)i; *H10K 85/60*(2023.01)i

FI: C07C211/63 CSP; C07C303/40; C07C311/48; H10K30/40; H10K30/50; H10K30/86; H10K50/135; H10K85/50; H10K85/60

According to International Patent Classification (IPC) or to both national classification and IPC

### B. FIELDS SEARCHED

Minimum documentation searched (classification system followed by classification symbols)

C07C211/63; C07C303/40; C07C311/48; H10K30/40; H10K30/50; H10K30/86; H10K50/135; H10K85/50; H10K85/60

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2020-113608 A (CENTRAL RESEARCH INSTITUTE OF ELECTRIC POWER INDUSTRY) 27 July 2020 (2020-07-27) paragraph [0126] | 1 |
| A | paragraph [0126] | 2-30 |
| X | WO 2022/010326 A1 (KOREA RESEARCH INSTITUTE OF CHEMICAL TECHNOLOGY) 13 January 2022 (2022-01-13) claims, paragraphs [61]-[62], [93]-[108], examples | 1-30 |
| P, X | KIM, Youngwoong et al., Alkylammonium bis(trifluoromethylsulfonyl)imide as a dopant in the hole-transporting layer for efficient and stable perovskite solar cells, Energy & Environmental Science, 29 March 2023, vol. 16, no. 5, pages 2226-2238 Broader context, fig. 1, experimental | 1-6, 14-30 |
| P, A | Broader context, fig. 1, experimental | 7-13 |

☑ Further documents are listed in the continuation of Box C.  ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **02 April 2024** | **23 April 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

## INTERNATIONAL SEARCH REPORT

International application No.

**PCT/JP2024/002706**

### C.    DOCUMENTS CONSIDERED TO BE RELEVANT

| Category* | Citation of document, with indication, where appropriate, of the relevant passages | Relevant to claim No. |
|---|---|---|
| P, X | NISHIMURA, Naoyuki et al., An Architype-Cation-Based Room Temperature Ionic Liquid: Aliphatic-Primary-Ammonium Bis(Trifluoromethylsulfonyl)Imide as An Additive for Hole Transport Layers in Perovskite Solar Cells Allowing Spontaneous Passivation of The Perovskite Layer, ChemRxiv [online], 06 March 2023, [retrieved on 18 March 2024], Retrieved from the Internet: <URL: https://chemrxiv.org/engage/chemrxiv/article-details/640472029789de3dd9f4c0d5> <br> 3. Results and discussion, Supporting information | 1-6, 14-15, 18-30 |
| P, A | 3. Results and discussion, Supporting information | 7-13, 16-17 |
| P, X | NISHIMURA, Naoyuki et al., Thermally Stable Phenylethylammonium-based Perovskite Passivation: Spontaneous Passivation with Phenylethylammonium Bis(trifluoromethylsulfonyl)imide under Deposition of PTAA for Enhancing Photovoltaic Performances of Perovskite Solar Cells, ChemRxiv [online], [retrieved on 18 March 2024], Retrieved from the Internet: <URL: https://chemrxiv.org/engage/chemrxiv/article-details/658d39b9e9ebbb4db9e71c80> <br> 3. Results and discussion, Supporting information | 1-9, 14-15, 18-30 |
| P, A | 3. Results and discussion, Supporting information | 10-13, 16-17 |
| P, X | NISHIMURA, Naoyuki et al., Archetype-Cation-Based Room-Temperature Ionic Liquid: Aliphatic Primary Ammonium Bis(trifluoromethylsulfonyl)imide as a Highly Functional Additive for a Hole Transport Material in Perovskite Solar Cells, ACS Applied Materials and Interfaces, 09 September 2023, vol. 15, no. 38, pages 44859-44866 <br> Results and discussion, Supporting information | 1-6, 14-15, 18-30 |
| P, A | Results and discussion, Supporting information | 7-13, 16-17 |
| A | WO 2022/128381 A1 (RHODIA OPERATIONS) 23 June 2022 (2022-06-23) <br> claims, examples | 1-30 |

Form PCT/ISA/210 (second sheet) (July 2022)

EP 4 660 180 A1

**INTERNATIONAL SEARCH REPORT**
Information on patent family members

International application No.

PCT/JP2024/002706

| Patent document cited in search report | | | Publication date (day/month/year) | Patent family member(s) | | | Publication date (day/month/year) |
|---|---|---|---|---|---|---|---|
| JP | 2020-113608 | A | 27 July 2020 | (Family: none) | | | |
| WO | 2022/010326 | A1 | 13 January 2022 | US | 2023/0178307 | A1 | |
| | | | | claims, paragraphs [0054]-[0055], [0082]-[0089], examples | | | |
| | | | | JP | 2023-517371 | A | |
| | | | | EP | 4181223 | A1 | |
| | | | | KR | 10-2022-0006750 | A | |
| | | | | CN | 115280532 | A | |
| WO | 2022/128381 | A1 | 23 June 2022 | EP | 4263427 | A1 | |
| | | | | CA | 3200173 | A1 | |
| | | | | CN | 116670069 | A | |
| | | | | KR | 10-2023-0118912 | A | |

Form PCT/ISA/210 (patent family annex) (July 2022)

**REFERENCES CITED IN THE DESCRIPTION**

**Patent documents cited in the description**

- WO 2021131428 A

- JP 2019096891 A

**Non-patent literature cited in the description**

- **P.V. KAMAT et al.** *J. Am. Chem. Soc.*, 2015, vol. 137, 1530-1538
- **JINBAO ZHANG et al.** *ACS Energy Lett.*, 2018, vol. 3, 1677-1682

- **SHEN WANG et al.** *Nano Lett.*, 2016, vol. 16, 5594-5600